(19) Europäisches Patentamt
European Patent Office
Office européen des brevets

(11) Publication number: **0 419 676 B1**

# (12) EUROPEAN PATENT SPECIFICATION

(45) Date of publication of patent specification: **07.12.94**

(51) Int. Cl.[5]: **C07C 317/22**, C07C 317/44, C07C 323/21, C07C 323/22, C07C 323/52, C07C 323/56, C07C 323/60, C07D 207/333, C07D 213/32, C07D 213/50

(21) Application number: **90905649.1**

(22) Date of filing: **30.03.90**

(86) International application number: **PCT/JP90/00441**

(87) International publication number: **WO 90/12001 (18.10.90 90/24)**

## (54) THIONAPHTHALENE DERIVATIVES, METHOD OF PRODUCING THE SAME, AND ANTIALLERGIC AGENT CONTAINING THE SAME.

(30) Priority: **30.03.89 JP 76730/89**

(43) Date of publication of application: **03.04.91 Bulletin 91/14**

(45) Publication of the grant of the patent: **07.12.94 Bulletin 94/49**

(84) Designated Contracting States: **AT BE CH DE DK ES FR GB IT LI NL SE**

(56) References cited:
**DE-A- 3 143 327**
**JP-A-63 270 634**

**TETRAHEDRON, August 1974, vol. 30, no. 16, Pergamon Press; Y. OIKAWA et al, "Cyclization of beta-ketosulfoxide-III, sythesis of naphthalene and phenanthrene derivatives", pp. 2653-2660**

(73) Proprietor: **TEIJIN LIMITED**
**6-7, Minamihonmachi 1-chome**
**Chuo-ku**
**Osaka-shi Osaka 541 (JP)**

(72) Inventor: **HAZATO, Atsuo**
**3-18-4-232, Tamadaira**
**Hino-shi**
**Tokyo 191 (JP)**
Inventor: **KATO, Yoshinori**
**7-24-11, Tamadaira**
**Hino-shi**
**Tokyo 191 (JP)**
Inventor: **KUROZUMI, Seizi**
**1-2-28, Higashitokura**
**Kokubunji-shi**
**Tokyo 185 (JP)**
Inventor: **OHMORI, Hitoshi**
**1-2-302, Tsushima-naka**
**Okayama-shi**
**Okayama 700 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, vol. 100, 1984, page 16, column 2, abstract no. 185314e, A. HAMADA et al, "Dopaminergic agonists: comparative actions of amine and sulfonium analogs of dopamine"

CHEMICAL ABSTRACTS, vol. 75, 1971, page 342, column 2, abstract no. 88363c, O. YONEMITSU et al, "Acid-catalyzed formation of naphthalenes from a beta-oxo sulfoxide"

Inventor: **MINOSHIMA, Toru**
**3-5-18, Tamadaira**
**Hino-shi**
**Tokyo 191 (JP)**
Inventor: **OSADA, Yoshio**
**3-5-18, Tamadaira**
**Hino-shi**
**Tokyo 191 (JP)**
Inventor: **NAGATA, Ikuo**
**2-7-12-201, Shinmei**
**Hino-shi**
**Tokyo 191 (JP)**
Inventor: **KOMORIYA, Keiji**
**1532-117, Uchikoshi-cho**
**Hachioji-shi**
**Tokyo 192 (JP)**

(74) Representative: **Harrison, Ivor Stanley et al**
**Withers & Rogers**
**4 Dyer's Buildings**
**Holborn**
**London EC1N 2JT (GB)**

## Description

The present invention relates to a thionaphthalene derivative, having an immunoglobulin E (hereinafter IgE) antibody production suppressive action, lipoxygenase suppressive action, and chemical mediator liberation inhibitory action, and useful as a therapeutical preparation for allergy symptoms such as brochnial asthma, allergic rhinitis, urticaria, anaphylaxy shock, atopic dermatitis, and hypersensitivity.

BACKGROUND ART

Anti-allergy drugs currently used for general purpose are aimed primarily at type I allergy diseases, due to the high frequency of the onset of type I allergies encountered by clinics. A large number of chemical mediators participate in the type I allergy, for example, histamine, serotonine, SRS-A, $LTB_4$ , prostaglandin, and PAF (platelet activating factor).

Most of these chemical mediators are released by the antigen-antibody reaction mediated by the IgE antibody.

More specifically, upon the penetration thereof, the antigen will stimulate T-cells or B-cells through macrophage, and due to such a stimulation, T-cells and B-cells are proliferated by mitosis, and the helper T-cells brought about by a differentiation of T-cells induce a differentiation of B-cells to antibody producing cells, whereby an IgE antibody is produced. This IgE is bound to the membrane surface of a mast cell or basophile to sensitize same. When an antigen penetration occurs at this position, it will be bound to a plurality of IgE antibodies bound on the membrane, whereby the IgE antibodies will be cross-linked. With this as the trigger, an influx of $Ca^{2+}$ into the cells occurs, and various enzymes, etc., are activated within a short time, to release the chemical mediators, and the allergy reaction is begun through the actions of these substances. Among these chemical mediators, as those which are inside granules and are released by degranulation, there are known histamine, ECF (eosinophil chemotactic factor), NCF (neutrophil chemotactic factor), natural protease (e.g., tryptase and carboxypeptidase $\beta$), exoglycoridase (e.g., arylsulfatase $\beta$, $\beta$-hexosaminidase, and $\beta$-glucuronidase) or proteoglycan, and these are called preformed mediators. On the other hand, as those contained in cell membranes and produced via various metabolism processes through antigen-antibody reactions, there are prostaglandins and PAF or metabolites of arachidonic acid such as $LTC_4$ , $D_4$ , and $E_4$ which are called SRS-A, $LTB_4$ and various HETE (hydroxyeicosatetraenoic acids), and these are called newly formed mediators. The symptoms of allergy diseases are considered to be exhibited through the complicated entanglements of these various chemical mediators.

Most of the antiallergy agents of the prior art may be classified broadly as drugs which, when these chemical mediators are discharged and released from the mast cells or basophiles, suppress the discharge and release thereof (e.g., Tranilast and DSCG [Disodium Cromoglicate]) and drugs which are antagonistic to chemical mediators, particularly histamine.

Nevertheless, since the allergy symptoms appear to be due to the complicated entanglements of the chemical mediators, the therapy presently practiced with only the drugs described above is limited, and thus various drugs having various new pharmaceutical effects have been developed. Among such drugs, as low molecular weight compounds, there may be included, for example, IgE antibody production suppressants, SRS-A antagonists, lipoxygenase suppressants, and PAF antagonists. The IgE antibody production inhibitors have been disclosed, for example, in Japanese Unexamined Patent Publications (Kokai) Nos. 62-53966, 59-167564, 59-170062, 60-152459, 64-83080, 1-149782, 1-135785, 1-290676, but none of these drugs is used in practice by clinics, and they are groups of compounds having an entirely different structure from the compounds of the present invention.

On the other hand, for the lipoxygenase inhibitor, compounds having a chemical mediator release suppressive activity as the primary pharmaceutical effect and a lipoxygenase inhibitory activity as the secondary pharmaceutical effect (e.g., Amlexanox and Azelastine) are in practical use by clinics, but no compound having a lipoxygenase inhibitory activity as the primary pharmaceutical effect is employed by clinics, and research and development is now underway in this field, by many pharmaceutical manufacturers. Also, no compound having an IgE antibody production suppressive action, lipoxygenase inhibitory action, and chemical mediator release suppressive action, at the same time, is known.

Therefore, the thionaphthalene derivative of the present invention is an entirely novel compound. Namely, although compounds similar to the thionaphthalene derivative of the present invention have been disclosed by V.N. Lisitsyn et al. (Zh. Org. Khim., 23 (9), 1942 (1987), A.M. Zeinalov et al. (Zh. Org. Khim., 15(4), 816 (1979), and A. Hamada et al. (J. Med. Chem., 1984, 27(5) 675), among others, no report has been made about effects such as an IgE antibody production suppressive activity, lipoxygenase inhibitory activity, and chemical mediator release suppressive activity exhibiting a usefulness as an anti-allergy agent,

in the compounds described in these literatures, and nothing has been disclosed from which the anti-allergy effect exhibited by the compound of the present invention could be easily estimated.

EP-A-0273678 discloses naphthalene derivatives which may include a thioester group attached via a long-chain methylene group to a thio moiety, as a substituent. The derivatives are intended to inhibit the biosynthesis of chemical mediators produced by lipoxygenase and cyclooxygenase.

The present inventors have made intensive efforts to find a compound different from the anti-allergy agent of the prior art, which will suppress the IgE antibody production upon the onset of an allergy, and further, has a lipoxygenase inhibitory activity and a chemical mediator release suppressive activity.

More specifically, in accordance with the present invention, there is provided a thionaphthalene derivative having the formula [I] shown below, or a non-toxic salt thereof, and an anti-allergy agent containing same as the active ingredient:

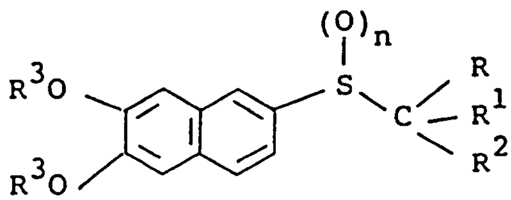

[I]

where R represents a hydrogen atom, an unsubstituted $C_1$ - $C_5$ alkyl group, a substituted or unsubstituted aryl group, a heterocyclic group, a group

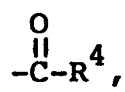

(where $R^4$ represents a substituted or unsubstituted $C_1$ - $C_4$ alkyl group, a substituted or unsubstituted aryl group or a heterocyclic group), a group

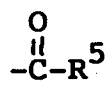

(where $R^5$ represents a hydrogen atom, a substituted or unsubstituted $C_1$ - $C_{10}$ alkyl group, a substituted or unsubstituted $C_3$ - $C_{10}$ alkenyl group or a substituted or unsubstituted $C_5$ - $C_7$ cycloalkyl group),
a group

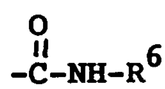

(where $R^6$ represents a substituted or unsubstituted $C_1$ - $C_4$ alkyl group, a substituted or unsubstituted aryl group or a heterocyclic group) or cyano group,

$R^1$ and $R^2$ each independently represent a hydrogen atom, a $C_1$ - $C_4$ alkyl group or phenyl group,

$R^3$ represents a hydrogen atom, a $C_1$ - $C_4$ alkyl group or

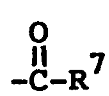

(where $R^7$ represents $-OR^{81}$, $-R^{82}$ or $-NR^{83}{}_2$, $R^{81}$, $R^{82}$ and $R^{83}$ each represents a $C_1$ - $C_4$ alkyl group), and

n represents an integer of 0 to 2, with the proviso that R, $R^1$ and $R^2$ cannot all represent H when n is O.

Examples of the unsubstituted $C_1$ - $C_5$ all of R include the methyl, ethyl, propyl, isopropyl, butyl, isobutyl, s-butyl, t-butyl, pentyl, isopentyl, neopentyl, and t-pentyl groups.

Examples of the unsubstituted aryl group of R include the phenyl, 1-naphthyl, and 2-naphthyl groups.

As the heterocyclic group of R, monocyclic or dicyclic groups having an oxygen, nitrogen or sulfur atom, such as the furyl, thienyl, pyrrolyl, oxazolyl, isooxazolyl, thiazolyl, isothiazolyl, imidazolyl, pyrazolyl, triazolyl, tetrazolyl, pyranyl, pyridyl, pyrazinyl, pyrimidinyl, benzofuranyl, indolyl, benzoimidazolyl, benzothiazolyl, benzooxazolyl, quinolyl, isoquinolyl, quinazolyl, purinyl, puteridinyl, morpholinyl, piperidinyl, and piperazinyl groups can be exemplified.

When R represents a

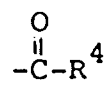

group, examples of the unsubstituted $C_1$ to $C_4$ alkyl group, the unsubstituted aryl group, and the unsubstituted heterocyclic group of $R^4$ include all of the groups exemplified above in the case of R.

When R represents

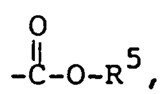

examples of the unsubstituted $C_1$ - $C_{10}$ alkyl group of $R^5$ are alkyl groups such as the methyl, ethyl, propyl, isopropyl, butyl, isobutyl, s-butyl, t-butyl, pentyl, isopentyl, neopentyl, t-pentyl, hexyl, heptyl, octyl, 3,7-dimethyloctyl, nonyl, and decyl groups, and the like, and examples of the unsubstituted $C_3$ - $C_{10}$ alkenyl groups are the 2-propenyl, 2-butenyl, 3-butenyl, 2-pentenyl, 4-pentenyl, 2-hexenyl, 3-hexenyl, 5-hexenyl, methallyl, citronelyl, and geranyl groups. As the unsubstituted $C_5$ to $C_7$ cycloalkyl group of $R^5$, the cyclopentyl, cyclohexyl, cycloheptyl groups are exemplified. When $R^5$ is a hydrogen atom, the carboxylic acid derivative can form a salt with a cationic compound. Namely, the acidic compound can be further allowed to react with an appropriate inorganic or organic salt to obtain a non-toxic salt purified therefrom. As such a base, the following compounds are exemplified. More specifically, as inorganic bases, hydroxides, carbonates, and bicarbonates of alkali metals, or alkaline earth metals such as sodium, potassium, calcium, and magnesium, and the like are exemplified. As organic bases, primary, secondary or tertiary alkylamines such as methylamine, dimethylamine, trimethylamine, ethylamine, diethylamine, and triethylamine, and the like; primary, secondary or tertiary alkanolamines such as ethanolamine, diethanolamine, triethanolamine, and 2-amino-2-hydroxymethyl-1,3-propane diol, and the like; diamines such as ethylenediamine and hexamethylenediamine, and the like; cyclic saturated or unsaturated amines such as pyrrolidine, piperidine, morpholine, piperazine, N-methylmorpholine, and pyridine, and others are exemplified.

When R represents

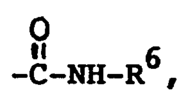

examples of the unsubstituted $C_1$ - $C_4$ alkyl group, the unsubstituted aryl group, and the heterocyclic group of $R^6$ include the groups exemplied above in the case of R.

When R is a substituted or unsubstituted aryl group, preferably the unsubstituted aryl group is a phenyl group. As the substituent, one or a plurality of halogen groups such as a chloro, bromo, fluoro group, trifluoromethyl group, methoxy group, cyano group, tetrazolyl group, and $-COOR^{91}$ group (carboxyl group, alkoxycarbonyl group) are included. In this case, examples of $R^{91}$ are those exemplified for $R^5$, and preferable examples thereof are the same as described below as preferable examples for $R^5$. The substituents mentioned here are preferably the chloro, fluoro, trifluoromethyl, methoxy, tetrazolyl, and methoxycarbonyl groups.

When R represents a heterocyclic group, as particularly preferably heterocyclic groups, the thienyl group, thiazolyl group, pyridyl group, imidazolyl group, tetrazolyl group, pyrrolyl group, pyrazinyl group, and piperadinyl group are included.

When R represents a

$$-\overset{O}{\overset{\|}{C}}-R^4$$

group, preferable examples of $R^4$, when $R^4$ is a substituted or unsubstituted $C_1$ - $C_4$ alkyl group, include linear groups such as the methyl, ethyl, propyl, and butyl groups as the unsubstituted alkyl group, and preferable substituents thereof include the hydroxy group, -$COOR^{92}$ (carboxyl group, alkoxycarbonyl group), substituted or unsubstituted aryl groups, and substituted or unsubstituted heterocyclic groups, but particularly preferable are the hydroxy group and -$COOR^{92}$ (carboxyl group, alkoxycarbonyl group). Here $R^{92}$ represents a hydrogen atom, a $C_1$ - $C_{10}$ substituted or unsubstituted alkyl group, a $C_3$ - $C_{10}$ substituted or unsubstituted alkenyl group, or a substituted or unsubstituted $C_5$ - $C_7$ cycloalkyl group. Examples of $R^{92}$ in this case include those exemplified in the case of $R^5$, and preferable examples thereof are the same as described below as preferable examples for $R^5$. This also applies to the non-toxic salts thereof.

When $R^4$ is a substituted or unsubstituted aryl group, as the unsubstituted aryl group, the phenyl group is preferable one. As a substituent thereof, one or a plurality of halogen groups such as the chloro, bromo, and fluoro groups, trifluoromethyl group, methoxy group, cyano group, tetrazolyl group, and -$COOR^{93}$ (carboxyl group, alkoxycarbonyl group) are included. Examples of $R^{93}$ in this case include those exemplified for $R^5$, and preferable examples thereof are the same as described below as preferable examples for $R^5$. Preferable substituents of those mentioned here include the chloro, fluoro, trifluoromethyl, methoxy, tetrazolyl, and methoxycarbonyl groups.

When $R^4$ represents a heterocyclic group, the preferable unsubstituted heterocyclic groups are the tienyl group, thiazolyl group, pyridyl group, imidazolyl group, tetrazolyl group, pyrrolyl group, pyrazinyl group, and piperadinyl group.

When R represents

$$-\overset{O}{\overset{\|}{C}}-O-R^5,$$

preferable examples of $R^5$, when $R^5$ is a substituted or unsubstituted $C_1$ - $C_{10}$ alkyl group, include linear alkyl groups such as the methyl, ethyl, propyl, butyl, and pentyl groups, or the isopropyl group and t-butyl group, and preferable substituents thereof include substituted or unsubstituted aryl groups, most particularly the phenyl group, a 4-alkyl-1-piperazinyl group, or a dialkylcarbamoyl group. In this case, there also may be a substituent on the phenyl group of the former, and as such a substituent, one or a plurality of halogen groups such as the chloro, bromo, and fluoro groups, and the trifluoromethyl group and methoxy group are preferable. In the case of a 4-alkyl-1-piperazinyl group, the alkyl group is preferably a methyl group, and the methyl group also may be substituted with one or two phenyl groups. There also may be a substituent on the phenyl group in this case, and examples thereof are the same as the phenyl group as described above.

When $R^5$ is a substituted or unsubstituted $C_3$ - $C_{10}$ alkenyl group, the 2-propenyl, 2-butenyl, methallyl, citronelyl, geranyl groups are included, and the substituent on the alkenyl group in this case includes halogen groups such as the fluoro, chloro, and bromo groups, the hydroxy group, acyloxy group, and alkoxy group, and among them, the chloro group, acetoxy group, and methoxy group are preferred.

When $R^5$ is a substituted or unsubstituted $C_5$ - $C_7$ cycloalkyl group, the cyclopentyl and cyclohexyl groups are included, and the substituent on the cycloalkyl includes the halogen groups such as the fluoro, chloro, and bromo groups, the hydroxy group, acyloxy group, and alkoxy group, and among them, the chloro group, acetoxy group, and methoxy group are preferred.

When $R^5$ is a hydrogen atom, the non-toxic cationic compound for forming a salt with the carboxylic acid preferably includes sodium, potassium, ethanolamine, triethanolamine, and 2-amino-2-hydroxymethyl-1,3-propanediol.

When R represents

$$-\overset{O}{\overset{\|}{C}}-NH-R^6,$$

preferable examples of $R^6$, when $R^6$ is a substituted or unsubstituted $C_1$ - $C_4$ alkyl group, include the linear methyl, ethyl, propyl, and butyl groups as the non-substituted alkyl group, and the preferable substituents thereof include substituted or unsubstituted aryl groups, most particularly the phenyl group or 4-alkyl-1-piperazinyl group. In this case, there also may be a substituent on the phenyl group of the former, and as the substituent, one or a plurality of halogen groups such as the chloro, bromo, and fluoro groups, and the trifluoromethyl group and methoxy group, are preferable. In the case of a 4-alkyl-1-piperazinyl group, the alkyl group is preferably the methyl group, and the methyl group also may be substituted with one or two phenyl groups. The phenyl group in this case also may have a substituent thereon, and examples thereof are the same as those of the phenyl group described above.

When $R^6$ is a substituted or unsubstituted aryl group, preferably the unsubstituted aryl group is a phenyl group, and as the substituent therefor includes one or a plurality of halogen groups such as the chloro, bromo, and fluoro groups, and the trifluoromethyl group, methoxy group, cyano group, tetrazolyl group, and -COOR$^{93}$ (carboxyl group, alkoxycarbonyl group). In this case, the examples of $R^{93}$ include those exemplified for $R^5$, and preferably, are the same as the preferable examples for $R^5$ described above.

When $R^6$ represents a heterocyclic group, preferably the heterocyclic group includes the thiazolyl group, pyridyl group, imidazolyl group, tetrazolyl group, and pyrazinyl group.

In the thionaphthalene derivative represented by the above formula [I], $R^1$ and $R^2$ are the same or different, and represent a hydrogen atom, a $C_1$ - $C_4$ alkyl group, or a phenyl group. Examples of the $C_1$ - $C_4$ alkyl groups are methyl, ethyl, propyl, isopropyl, butyl, isobutyl, and s-butyl and the like, and among them, the methyl group is preferred. Particularly, when R is other than

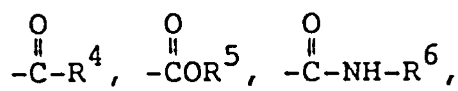

$R^1$ and $R^2$ are preferably both hydrogen atoms. On the other hand, when R is

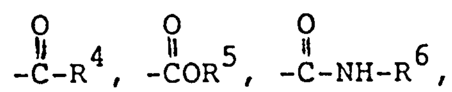

$R^1$ and $R^2$ may be the same or different, and are preferably groups selected from a hydrogen atom, a methyl group, and a phenyl group, and among them, preferably $R^1$ and $R^2$ are both hydrogen atoms, a hydrogen atom and a methyl group, are both methyl groups, or are a hydrogen atom and a phenyl group.

In the thionaphthalene derivative represented by the above formula [I], $R^3$ represents a hydrogen atom, a $C_1$ - $C_4$ alkyl group or

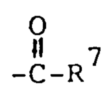

(where $R^7$ represents -OR$^{81}$, -R$^{82}$ or -NR$_2^{83}$, and $R^{81}$, $R^{82}$, and $R^{83}$ each represent a $C_1$ - $C_4$ alkyl group). The alkyl group of $C_1$ - $C_4$ in $R^3$, $R^{81}$, $R^{82}$, and $R^{83}$ includes the methyl, ethyl, propyl, isopropyl, butyl, isobutyl, and s-butyl groups, and among them, the methyl group is preferred. Preferable examples of $R^3$ are a hydrogen atom, a methyl group, an acetyl group, a methoxycarbonyl group, and a dimethylcarbamoyl group.

Specific examples of such thionaphthalene derivatives include the following compounds.

| | |
|---|---|
| I-(1) | 2-(Methylthio)-6,7-dihydroxynaphthalene |
| I-(2) | 2-(Ethylthio)-6,7-dihydroxynaphthalene |
| I-(3) | 2-(Propylthio)-6,7-dihydroxynaphthalene |
| I-(4) | 2-(Butylthio)-6,7-dihydroxynaphthalene |
| I-(5) | 2-(Pentylthio)-6,7-dihydroxynaphthalene |
| I-(6) | 2-(Isopropylthio)-6,7-dihydroxynaphthalene |
| I-(7) | Methyl 3-(6,7-dihydroxy-2-naphthylthio)propionate |
| I-(8) | 3-(6,7-dihydroxy-2-naphthylthio)ethyl alcohol |
| II-(1) | 2-(Benzylthio)-6,7-dihydroxynaphthalene |

II-(2) 2-(2-Pyridylmethylthio)-6,7-dihydroxynaphthalene
II-(3) 2-(Pirazinylmethylthio)-6,7-dihydroxynaphthalene
II-(4) 2-(Thienylmethylthio)-6,7-dihydroxynaphthalene
II-(5) 2-(Thiazolinylmethylthio)-6,7-dihydroxynaphthalene
II-(6) 2-(Tetrazolylmethylthio)-6,7-dihydroxynaphthalene
III-(1) 1-(6,7-dihydroxy-2-naphthylthio)-2-butanone
III-(2) 1-(6,7-dihydroxy-2-naphthylthio)-5-methoxycarbonyl-2-pentanone
III-(3) 2-(6,7-dihydroxy-2-naphthylthio)benzophenone
III-(4) (6,7-dihydroxy-2-naphthylthio)methyl-2-pyridyl ketone
III-(5) (6,7-dihydroxy-2-naphthylthio)methyl pyrazinyl ketone
III-(6) (6,7-dihydroxy-2-naphthylthio)methyl-2-pyrrolyl ketone
III-(7) (6,7-dihydroxy-2-naphthylthio)methyl-2-thiazolyl ketone
III-(8) (6,7-dihydroxy-2-naphthylthio)methyl-2-thienyl ketone
IV-(1) Methyl (6,7-dihydroxy-2-naphthylthio)acetate
IV-(2) (6,7-Dihydroxy-2-naphthylthio)acetic acid
IV-(3) Sodium salt of (2)
IV-(4) Potassium salt of (2)
IV-(5) Triethanolamine salt of (2)
IV-(6) Ethyl (6,7-dihydroxy-2-naphthylthio)acetate
IV-(7) Butyl (6,7-dihydroxy-2-naphthylthio)acetate
IV-(8) Isopropyl (6,7-dihydroxy-2-naphthylthio)acetate
IV-(9) Benzyl (6,7-dihydroxy-2-naphthylthio)acetate
IV-(10) Methallyl (6,7-dihydroxy-2-naphthylthio)acetate
IV-(11) Cyclohexyl (6,7-dihydroxy-2-naphthylthio)acetate
IV-(12) Geranyl (6,7-dihydroxy-2-naphthylthio)acetate
IV-(13) (3,7-Dimethyl)-6-octenyl (6,7-dihydroxy-2-naphthylthio)acetate
IV-(14) (5-Phenyl)pentyl (6,7-dihydroxy-2-naphthylthio)acetate
IV-(15) 2-Amino-2-hydroxymethyl-1,3-propane diol salt of (2)
IV-(16) Dimethylcarbamoylmethyl (6,7-dihydroxy-2-naphthylthio)acetate
IV-(17) t-Butyl (6,7-dihydroxy-2-naphthylthio)acetate
V-(1) N-butyl(6,7-dihydroxy-2-naphthylthio)acetamide
V-(2) N-5-tetrazolyl(6,7-dihydroxy-2-naphthylthio)-acetamide
V-(3) N-(2-carboxyphenyl)(6,7-dihydroxy-2-naphthylthio)acetamide
V-(4) N-(2-methoxycarbonylphenyl)(6,7-dihydroxy-2-naphthylthio)acetamide
V-(5) N-[2-(5-tetrazolyl)phenyl](6,7-dihydroxy-2-naphthylthio)acetamide
V-(6) 1-[4-[(6,7-dihydroxy-2-naphthylthio)acetylamino]-butyl]-4-[bis(4-fluorophenyl)methyl]piperazine
VI-(1) Methyl (6,7-dimethoxy-2-naphthylthio)acetate
VI-(2) Methyl (6,7-diacetoxy-2-naphthyithio)acetate
VI-(3) Methyl (6,7-dimethoxycarbonyloxy-2-naphthylthio)acetate
VI-(4) Methyl (6,7-diisopropoxycarbonyloxy-2-naphthylthio)acetate
VI-(5) Methyl [6,7-bis(dimethylcarbamoyloxy)-2-naphthylthio]acetate
VII-(1) Methyl 2-(6,7-dihydroxy-2-naphthylthio)-propionate
VII-(2) Methyl 2-methyl-2-(6,7-dihydroxy-2-naphthylthio)propionate
VII-(3) Methyl 2-(6,7-dihydroxy-2-naphthylthio)phenylacetate
VIII-(1) 2-(Benzylsulfonyl)-6,7-dihydroxynaphthalene
VIII-(2) 2-(Benzylsulfinyl)-6,7-dihydroxynaphthalene
VIII-(3) Methyl (6,7-dihydroxy-2-naphthylsulfinyl) acetate
IX-(1) (6,7-Dihydroxy-2-naphthylthio)acetonitrile.

Further, according to the present invention, there is provided a method of preparing a thionaphthalene derivative having the formula [I']:

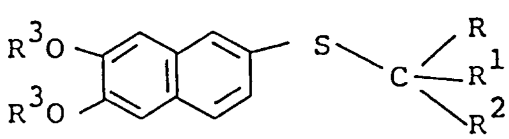

[I']

wherein R, $R^1$, $R^2$ and $R^3$ are as defined above, which comprises reacting a thiol having the formula [II]:

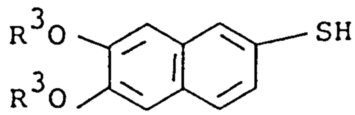

[II]

wherein $R^3$ is as defined above, with a halide compound having the formula [III]:

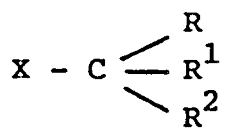

wherein X represents a halogen atom, and R, $R^1$ and $R^2$ are as defined above, in the presence of a basic compound.

Furthermore, according to the present invention, there is provided a method of preparing a thionaphthalene derivative having the formula [I"]:

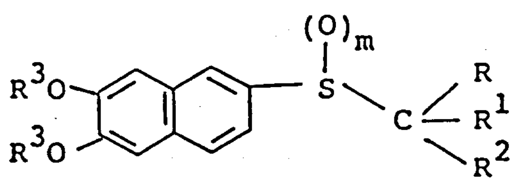

[I"]

wherein R, $R^1$, $R^2$, and $R^3$ are as defined above and m represents 1 or 2, which comprises reacting a thionaphthalene derivative having the formula [I']:

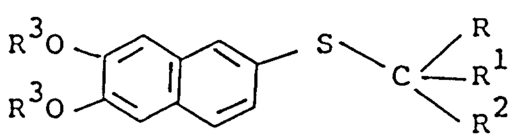

[I']

wherein R, $R^1$, $R^2$ and $R^3$ are as defined above with an oxidizing agent.

The thionaphthalene derivative of the present invention having the above formula [I] can be obtained by allowing a compound having the above formula [II] to react with a compound having the above formula [III], in the presence of a basic compound.

The reaction between the compound of the above formula [II] and the compound of the above formula [III] can be carried by an anionization of the compound [II] with a basic compound such as NaH or $CH_3ONa$, or by reacting a mixture of the compound of the above formula [II] and the compound of the above formula [III] in an organic base, such as pyridine, triethylamine, and DBU. In this case, as the solvent to be used in the reaction, for example, tetrahydrofuran (THF), dimethylformamide, diethyl ether, and dioxane may be employed or only the organic base as mentioned above may be employed as the solvent.

Preferably, the base such as NaH and $CH_3ONa$ is employed in an amount of 0.5 to 10-fold equivalents, more preferably 1 mole equivalent stoichiometrically, based on the thiol compound [II]. Preferably, the compound having the above formula [III] is employed in an amount of 0.1 to 5-fold equivalents, more preferably 0.7 to 1.5-fold equivalents, based on the thiol compound. Preferably, the reaction temperature is from -30°C to 200°C, more preferably from 0 to 100°C, and the reaction time is preferably from 10 minutes to 100 hours, more preferably from 1 hour to 24 hours. After completion of the reaction, the thionaphthalene derivative having the above [I'] can be obtained by a conventional post-treatment such as extraction or column chromatography.

The thionaphthalene derivative [I'] then can be subjected to the oxidation reaction, to be converted to the corresponding sulfone or sulfoxide derivative [I"].

As the oxidizing agent to be used when preparing a sulfoxide, for example, preferably peracids such as hydrogen peroxide, peracetic acid, perbenzoic acid, and m-chloroperbenzoic acid and the like; and sodium

metaperiodate, hydroperoxide, selenium dioxide, chromic acid, iodosylbenzene, hypochloric acid, and t-butyl hydroperoxide, are employed. When preparing sulfone, preferably for example, hydrogen peroxide, hydrogen peroxide and tungsten or vanadium catalyst, peracetic acid, perbenzoic acid, m-chloroperbenzoic acid, ruthenium oxide, and osmium oxide [VIII] are employed.

When $R^3$ is hydrogen in [I'], the phenolic hydroxyl group also may be oxidized, and therefore, preferably a peracid such as hydrogen peroxide or m-chloroperbenzoic acid is used.

As the reaction solvent, for example, water, acetic acid, methanol, methylene chloride, chloroform, 1,2-dichloroethane, benzene, and ethyl acetate, or solvent mixtures thereof, are preferably employed.

The reaction temperature is preferably from -78°C to 50°C, more preferably from -20°C to 30°C.

The reaction time depends on the starting compounds, the reaction temperature, and the oxidizing agent employed, but is preferably 30 minutes to 48 hours.

For example, when a sulfoxide is to be prepared by using an oxidizing agent which can prepare both sulfoxide and sulfone, preferably the amount of the oxidizing agent is insufficient to give sulfone, for example, is about 1 to about 1.5 equivalents based on the compound employed, and the reaction is monitored by TLC.

The desired product after completion of the reaction can be separated and purified according to conventional methods, for example, by a combination of methods such as filtration concentration and chromatography.

The thiol derivative [II] to be used in the preparation method of the present invention is disclosed in Japanese Unexamined Patent Publication (Kokai) No. 63-270634, and can be synthesized according to the route shown below.

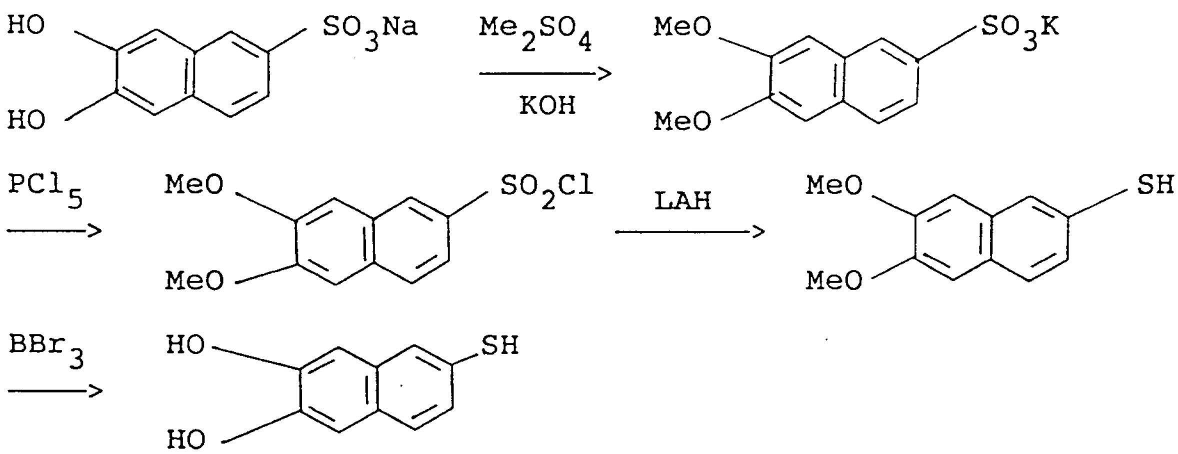


Surprisingly, the thionaphthalene derivative having the above formula [I] thus-obtained has a specific suppressive action for IgE production; for example, it has been clarified by the present inventors that it can inhibit the anti-TNP-IgE production of mouse spleen cells immunized with TNP-KLH (trinitropenyl-key hole limpet hemocyanine). Note, the anti-TNP-IgG production was not substantially suppressed.

Therefore, the compound of the present invention can suppress the production of an antigen specific IgE induced after the antigen stimulation, to thereby finally suppress the release of the chemical mediator released from mast cells or basophiles by the antigenantibody reaction mediated by IgE.

It has been clarified that the thionaphthalene derivative having the above formula [I] in the present invention acts directly on mast cells, thereby suppressing the release of chemical mediators from the mast cells, particularly histamine. Further, it has been clarified that the thionaphthalere derivative represented by the above formula [I] in the present invention exhibits an inhibitory activity for lipoxygenase, thereby inhibiting a production from leukocytes such as leukotrienes $C_4$, $D_4$, and $E_4$, known as SRS-A, and leucotriene $B_4$ and 5-HETE.

Thus, the thionaphthalene derivative of the present invention has the actions of an IgE antibody production suppression, lipoxygenase inhibition, and suppression of chemical mediator liberation from mast cells, and therefore, is useful for the therapy or prophylaxis of diseases such as allergy diseases, including bronchial asthma, nasal allergy, ophthalmia allergy, and atopic dermatitis.

For the above-mentioned purpose, the thionaphthalene derivative of the present invention can be administered orally or parenterally, for example, by an intrarectal, subcutaneous, intramuscular, intravenous, percutaneous administration, or by inhalation.

A solid preparation or a liquid preparation thereof can be used for an oral administration, and as solid preparation, for example, tablets, pills, powders or granules can be used. In such solid preparations, one or more active substance is mixed with at least one pharmaceutically acceptable carrier, such as sodium bicarbonate, calcium carbonate, potato starch, sucrose, mannitol, or carboxymethyl cellulose. The preparation operation may be practiced in a conventional manner, but in addition to those mentioned above, additives for the preparation, for example, lubricants such as calcium stearate, magnesium stearate, and glycerine also may be contained.

The liquid preparation for the oral administration contains, for example, an emulsion, a solution, a suspension, a syrup or an elexir, and these preparations may contain pharmaceutically acceptable carriers as generally employed, such as water or liquid paraffin.

Oily bases such as coconut oil, resolved coconut oil, soybean oil, and corn oil, also can be used as the carrier.

For the oral administration, the preparation, for example, the above-mentioned solid preparation, can be applied by blowing with coating of a solution of an enteric soluble substance such as cellulose acetate phthalate, hydroxypropyl methyl cellulose phthalate, polyvinyl alcohol phthalate, styrene-maleic anhydride copolymer or methacrylic acid, methyl methacrylate copolymer in an organic solvent or water, to be formed into an enteric soluble preparation. An enteric soluble preparation such as powders or granules also can be contained in the capsules.

The pharmaceutically acceptable carrier also may include the auxiliary agents, aromatics, stabilizers or preservatives usually employed, if necessary.

Also, the liquid preparation may be administered in a capsule made of an absorbable substance such as gelatin.

As the solid preparation for an intrarectal administration, suppositories containing one or more active substance and prepared according to the method known per se can be used.

The preparation for the parenteral administration is given as a sterile aqueous or non-aqueous agent, suspension, or emulsion. The non-aqueous solution or suspension employs, for example, a propylene glycol, a polyethylene glycol, or a vegetable oil such as olive oil, and an injectable organic ester such as ethyl oleate as the pharmaceutically acceptable carrier. This preparation also can contain auxiliary agents such as a preservative, humectant, emulsifier, dispersing agent, and stabilizer. These solutions, suspensions and emulsions can be sterilized by, for example, filtration, by passing them through a bacteria retaining filter, formulation with a sterilizer, or an application of a treatment such as irradiation. Also, a sterile solid preparation can be prepared and dissolved in a sterile water or a sterile solvent for injection, immediately before use.

Also, for an inhalation, a solution or suspension of the compound of the present invention with a conventionally used excipient may be employed, for example, as an aerosol spray for inhalation. Also, the compound can be administered by an inhalator or other means which enables a direct contact with the lungs of the active compound in the form of a dry powder.

As the dosage form for a percutaneous administration, for example, ointments may be used.

The dose of the aromatic derivative of the present invention depends on the condition, the age, the sex, the body weight, and the rote of administration, etc., of the subject receiving the administration, but can be administered at a dose of about 0.1 mg to 1000 mg/kg-body weight/day. Such a dose can be administered once or several times, for example, in divided doses 2 to 6 times per day.

## Examples

The present invention is now described in more detail with reference to Examples, but it should be noted that the scope of the present invention is not limited to these Examples.

Example 1

Synthesis of 2-(methylthio)-6,7-dihydroxynaphthalene

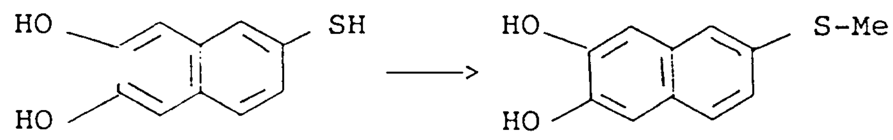

I-(1)

To a 6 ml methanolic solution of 202 mg (1.05 mmol) of 6,7-dihydroxy-2-mercaptonaphthalene was added a methanolic solution of 220 mg (1.05 mmol) of MeONa (28% in MeOH) under a nitrogen atmosphere, and the mixture was stirred at room temperature for 20 minutes. The mixture was ice-cooled, 66 μl (1.05 mmol) of methyl iodide then added, and the mixture stirred at room temperature for 10 hours. Water was added to complete the reaction, and the mixture was extracted with ethyl acetate. The organic layer was washed with saturated sodium chloride and dried over anhydrous magnesium sulfate, and ethyl acetate was evaporated under a reduced pressure to give 147 mg (68%) of the crude product.

$^1$H-NMR (90 MHz, δ ppm, $CDCl_3$);
2.53 (s, 3H), 7.0 - 7.7 (m, 5H)

Example 2

Synthesis of 2-butylthio-6,7-dihydroxynaphthalene

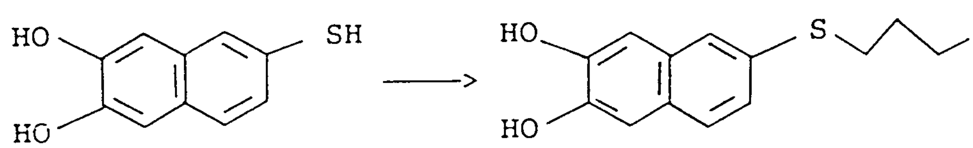

I-(4)

To a 6 ml methanolic solution of 208 mg (1.08 mmol) of 6,7-dihydroxy-2-mercaptonaphthalene was added a methanolic solution of 220 mg (1.05 mmol) of MeONa (28% in MeOH) under a nitrogen atmosphere, and the mixture was stirred at room temperature for 20 minutes. Subsequently, 2 ml of a methanolic solution of 156 mg (1.05 mmol) of n-butyl bromide was added, the mixture was stirred at room temperature for 10 hours, water was added to complete the reaction, and the mixture was extracted with ethyl acetate. The organic layer was washed with saturated sodium chloride and dried over anhydrous magnesium sulfate, and ethyl acetate was evaporated under a reduced pressure to give 259 mg (95%) of the crude product. Recrystallization was carried out from benzene-hexane.

mp; 123 - 126 °C
$^1$H-NMR (δ ppm, $(CD_3)_2CO$);
0.90 (t, 3H, J = 7 Hz) 1.2 - 1.8 (m, 4H)
2.96 (t, 2H, J = 7 Hz) 7.1 - 7.3 (m, 3H)
7.5 - 7.7 (m, 2H). 8.37 (s, 2H)
IR (KBr, $cm^{-1}$); 3400, 2960, 2930, 1520, 1420 1260, 1150, 1110

Example 3

Synthesis of methyl 3-(6,7-dihydroxy-2-naphthylthio)propionate

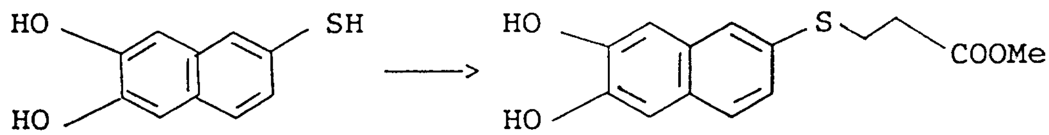

I-(7)

First, 2 ml of methanolic solution of 242 mg (1.26 mmol) of 6,7-dihydroxy-2-mercaptonaphthalene was cooled to 0°C under a nitrogen atmosphere, a drop of MeONa (28% in MeOH) (about 7 mg, 0.04 mmol) was added thereto and 125 μl (1.39 mmol, 1.1 equivalent) of methyl acrylate then added, followed by stirring at room temperature for 4 hours. After the reaction, ether and saturated aqueous potassium hydrogen sulfate were added, the mixture was extracted with ether, and the organic layer was washed with saturated aqueous sodium chloride and dried over anhydrous magnesium sulfate. Ether was evaporated under a reduced pressure to give 350 mg of the crude product. The product was recrystallized from chloroform to give 230 mg (64%) of methyl 3-(6,7-dihydroxy-2-naphthylthio)propionate.

mp; 125 - 127°C

$^1$H-NMR (90 MHz, δ ppm, $CDCl_3$);

2.62 (t, 2H, J = 7.0 Hz) 3.20 (t, 2H, J = 7.0 Hz)

3.60 (s, 3H) 7.1 - 7.35 (m, 3H)

7.45 - 7.75 (m, 3H) 8.42 (br. s, 2H)

Example 4

Synthesis of 2-(6,7-dihydroxy-2-naphthylthio)ethanol

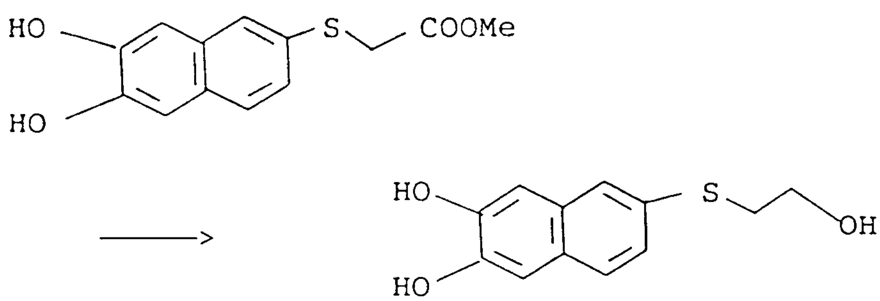

I-(8)

An amount of 224 mg of methyl (6,7-dihydroxy-2-naphthylthio)acetate was dissolved in 5 ml of tetrahydrofuran, and to the solution was added 35 mg of lithium aluminum hydride at 0°C, and the mixture stirred for 2 hours.

To the reaction mixture was added 3N hydrochloric acid to deactivate the reducing agent, and the desired product was extracted from the reaction mixture with ethyl acetate. The organic layer obtained was washed with saturated aqueous sodium chloride, and dried over anhydrous magnesium sulfate, and the solution was concentrated under a reduced pressure. The desired product was separated by column chromatography, and further crystallized from ether and chloroform to give 162 mg (81%) of 2-(6,7-dihydroxy-2-naphthylthio)ethanol.

$^1$H-NMR (90 MHz, δ ppm, $d_6$-Acetone);

3.09 (t, J = 6.8 Hz, 2H) 3.71 (t, J = 6.8 Hz, 2H)

7.1 - 7.7 (m, 5H)

Example 5

Synthesis of 2-(benzylthio)-6,7-dihydroxynaphthalene

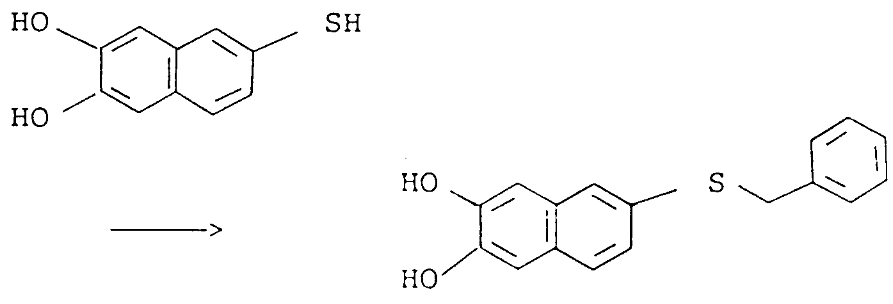

II-(1)

An amount of 216 mg of 6,7-dihydroxy-2-mercaptonaphthalene was dissolved in 1 ml of methanol, and to the solution were added 253 µl of 28% sodium methoxidemethanol solution and 147 µl of benzyl bromide, and the mixture was stirred for 6 hours.

The reaction mixture was poured into water, and the desired product extracted therefrom with ethyl acetate. The organic layer obtained was washed with saturated aqueous sodium chloride and dried over anhydrous magnesium sulfate, and the solution was concentrated under a reduced pressure. The desired product was separated by column chromatography, and further, crystallized from ether, chloroform, and hexane to give 162 mg (65%) of 2-(benzylthio)-6,7-dihydroxynaphthalene.

$^1$H-NMR (90 MHz, δ ppm, $d_6$-Acetone);

4.21 (s, 2H) 7.1 - 7.3 (m, 10H)

Example 6

Synthesis of 2-(2-pyridylmethylthio)-6,7-dihydroxynaphthalene

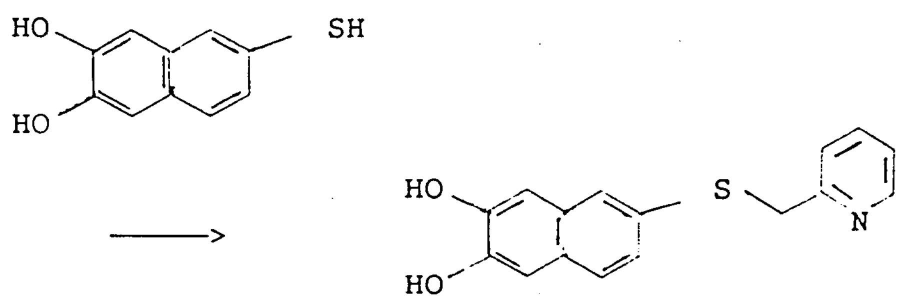

II-(2)

An amount of 268 mg of 6,7-dihydroxy-2-mercaptonaphthalene was dissolved in 1.5 ml of methanol, and to the solution were added 627 µl of 28% sodium methoxidemethanol solution at room temperature and 252 mg of 2-picoryl chloride hydrochloride, and the mixture was stirred for 6 hours.

The reaction mixture was poured into water, and the desired product extracted therefrom with ethyl acetate. The organic layer obtained was washed with saturated aqueous sodium chloride and dried over anhydrous magnesium sulfate, and the solution was concentrated under a reduced pressure. The desired product was separated by column chromatography, and crystallized from ether, chloroform, and hexane to give 182 mg (46%) of 2-(2-pyridylmethylthio)-6,7-dihydroxynaphthalene.

$^1$H-NMR (90 MHz, δ ppm, $d_6$-Acetone);

4.31 (s, 2H) 7.0 - 7.8 (m, 9H)

Example 7

Synthesis of 2-(tetrazolylmethylthio)-6,7-dihydroxynaphthalene

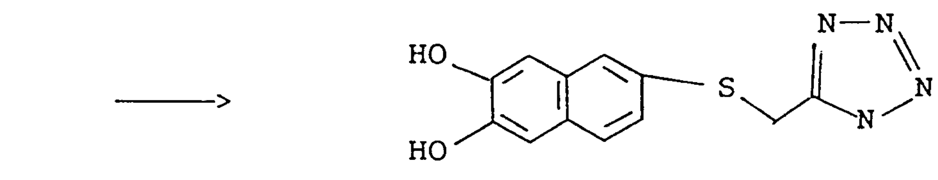

II-(6)

An amount of 289 mg of (6,7-dihydroxynaphthylthio)acetonitrile and 406 mg of sodium azide were dissolved in 4 ml of dimethylformamide, and the reaction mixture was stirred at 120 ° C for 5 hours.

The reaction mixture was cooled to room temperature, and then neutralized with 3N hydrochloric acid, and the desired product was extracted from the mixture with ethyl acetate. The organic layer obtained was washed with saturated aqueous sodium chloride and dried over anhydrous magnesium sulfate, and the solution was concentrated under a reduced pressure. The product was purified by column chromatography to give 342 mg (100%) of 2-(tetrazolylmethylthio)-6,7-dihydroxynaphthalene.

$^1$H-NMR (90 MHz, δ ppm, $d_6$-Acetone);

3.32 (s, 2H) 4.51 (s, 2H) 7.1 - 8.0 (m, 5H)

Example 8

Synthesis of 1-(6,7-dihydroxy-2-naphthylthio)-2-butanone

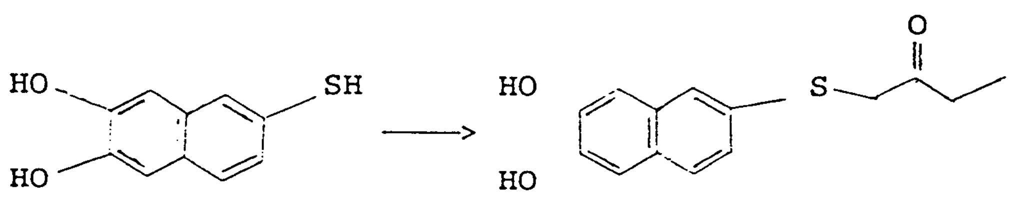

III-(1)

A 2 ml pyridine solution of 100 mg (0.52 mmol) of 6,7-dihydroxy-2-mercaptonaphthalene was ice-cooled, 58 μl (0.57 mmol of 1-bromo-2-butanone was added dropwise thereto, and subsequently, the mixture was stirred at room temperature for 1.5 hours. To the reaction mixture was added saturated aqueous potassium hydrogen sulfate, and the mixture was extracted with ether. The organic layer as washed with saturated potassium hydrogen sulfate, water, and saturated aqueous sodium chloride, and dried over anhydrous magnesium sulfate. The solvent was evaporated under a reduced pressure, and the oily product obtained was subjected to Florisil® SEP-PAK® (ether) to give 130 mg of the crude product. Recrystallization from chloroform gave 85 mg (62%) of 1-(6,7-dihydroxy-2-naphthylthio)-2-butanone as colorless crystals.

mp; 113 - 115 ° C

$^1$H-NMR (90 MHz, δ ppm, $CDCl_3$);

1.00 (t, 3H, J = 7.5 Hz) 2.65 (dd, 2H, J = 6.0 Hz)

3.85 (s, 2H) 7.6 (s, 1H) 8.44 (br. s, 2H)

Example 9

Synthesis of 1-(6,7-dihydroxy-2-naphthylthio)-5-methoxycarbonyl-2-pentanone

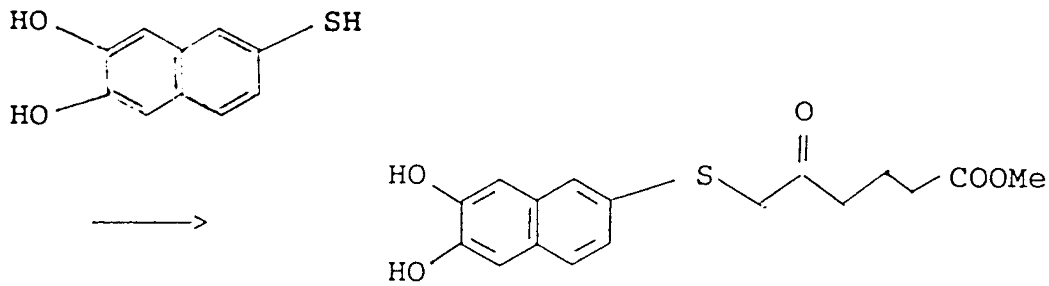

III-(2)

A 3 ml pyridine solution of 203 mg (1.05 mmol) of 6,7-dihydroxy-2-mercaptonaphthalene was ice-cooled, a 2 ml pyridine solution of 284 mg (1.27 mmol) of 1-bromo-5-methoxycarbonyl-2-pentanone was added dropwise thereto, and subsequently, after ice-cooling for 30 minutes, the mixture was stirred at room temperature overnight. The solution was neutralized with dilute hydrochloric acid and extracted with ethyl acetate, and the organic layer was washed with dilute hydrochloric acid, saturated sodium hydrogen carbonate solution, and saturated aqueous sodium chloride, and dried over anhydrous magnesium sulfate. The solvent was evaporated under a reduced pressure, and the oily product obtained was subjected to silica gel chromatography (hexane:ethyl acetate = 9:1) to give 173 mg (49%) of 1-(6,7-dihydroxy-2-naphthylthio)-5-methoxycarbonyl-2-pentanone.

$^1$H-NMR (90 MHz, δ ppm, $(CD_3)_2CO$);
1.84 (tt, J = 7.1 Hz, 6.8 Hz)
2.28 (t, 2H, J = 7.1 Hz)
2.72 (t, 2H, J = 6.8 Hz) 2.60 - 3.30 (br. s, 2H)
3.58 (s, 3H) 3.84 (s, 2H) 7.11 - 7.61 (m, 5H)

Example 10

Synthesis of 2-(6,7-dihydroxy-2-naphthylthio)benzophenone

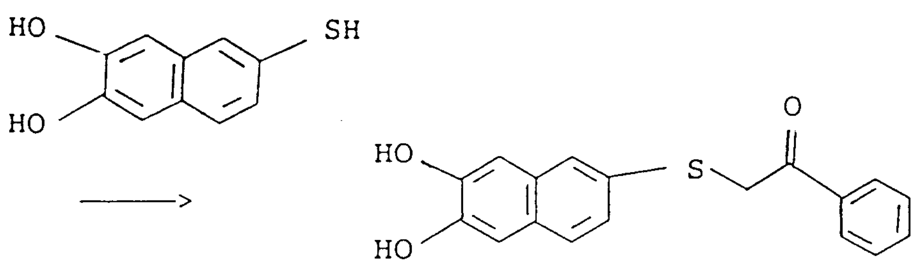

III-(3)

A 3 ml pyridine solution of 221 mg (1.15 mmol) of 6,7-dihydroxy-2-mercaptonaphthalene was added under ice-cooling to a 3 ml methylene chloride solution of 229 mg (1.15 mmol) of 2-bromoacetophenone, and the mixture was stirred at room temperature for 4 hours. The reaction was completed with an aqueous potassium hydrogen sulfate, the mixture was extracted with ethyl acetate, and the extract was washed with water and saturated aqueous sodium chloride, and dried over anhydrous magnesium sulfate. The solvent was evaporated under a reduced pressure, and the residue subjected to silica gel column chromatography

(hexane:ethyl acetate = 2:1 - 1:1) to give 271 mg (76%) of 2-(6,7-dihydroxy-2-naphthylthio)benzophenone.

$^1$H-NMR (90 MHz, $\delta$ ppm, $CDCl_3$);
4.48 (s, 2H) 7.13 - 7.70 (m, 8H)
7.95 - 8.10 (m, 2H) 8.45 (br. s, 2H)

Example 11

Synthesis of (6,7-dihydroxy-2-naphthylthio)methyl-2-pyridil ketone

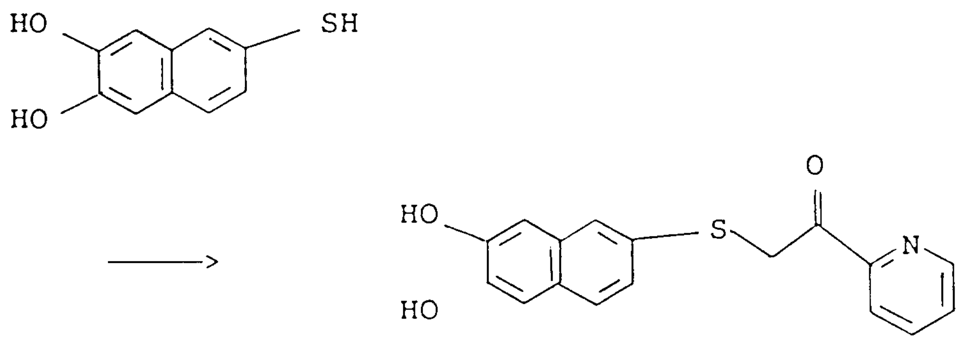

III-(4)

A 2 ml amount of 2-acetylpyridine was dissolved in 15 ml of acetic acid, the solution was added to 1.01 ml of bromine at room temperature, and the mixture was stirred at 90 ° C for 5 hours.

After a removal of acetic acid from the reaction mixture under a reduced pressure, a saturated aqueous sodium hydrogen carbonate was added until the pH of the solution became about 9, and the desired product was extracted from the mixture with ethyl acetate. The organic layer obtained was washed with saturated aqueous sodium chloride, and dried over anhydrous magnesium sulfate, the solution was concentrated under a reduced pressure, and the residue distilled (about 150 ° C/20 mmHg) to give about 2 g (ca. 28%) of 2-($\alpha$-bromoacetyl)pyridine.

$^1$H-NMR (90 MHz, $\delta$ ppm, $CDCl_3$);
4.85 (s, 2H)
7.51 (ddd, J = 1.5, 4.7 & 7.4 Hz, 1H)
7.86 (dt, J = 1.7 & 7.6 Hz, 1H)
8.10 (d, J = 7.9 Hz, 1H)
8.69 (d, J = 4.8 Hz, 1H)

A 221 mg amount of 6,7-dihydroxy-2-mercaptonaphthalene was dissolved in 15 ml of methylene chloride, and to the solution was added, at room temperature, a solution of 476 mg of 2-($\alpha$-bromoacetyl)-pyridine obtained above in methylene chloride (3 ml), and the mixture stirred for 3 hours.

To the reaction mixture was added saturated aqueous sodium hydrogen carbonate, the desired product was extracted from the mixture with ethyl acetate, the organic layer obtained was washed with saturated aqueous sodium chloride and dried over anhydrous magnesium sulfate, and the solution was concentrated under a reduced pressure. To the concentrate was added methylene chloride, and insolubles in methylene chloride then removed by filtration. The filtrate was again concentrated under a reduced pressure, and the concentrate was purified by column chromatography to give 228 mg (74%) of (6,7-dihydroxy-2-naphthylthio)methyl 2-pyridil ketone.

$^1$H-NMR (90 MHz, $\delta$ ppm, $d_6$-Acetone);
4.58 (s, 2H) 7.15 (d, J = 3.7 Hz, 2H)
7.2 - 7.7 (m, 4H) 7.98 (d, J = 4.8 Hz, 2H)
8.68 (d, J = 4.6 Hz, 1H)

Example 12

Synthesis of (6,7-dihydroxy-2-naphthylthio)methyl-2-pyrazinyl ketone

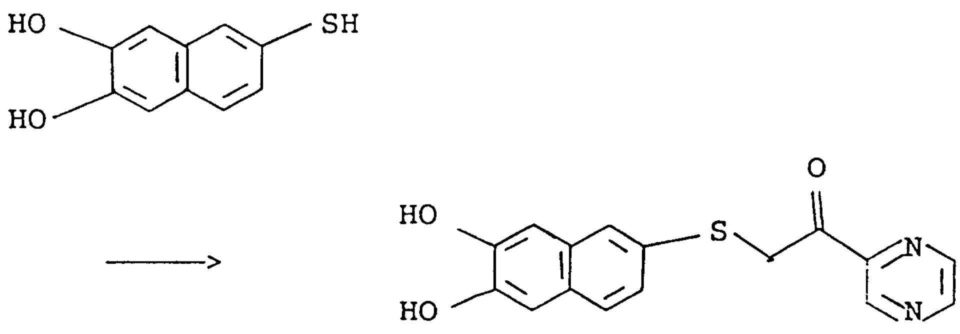

III-(5)

A 1.07 g amount of acetylpyrazine was dissolved in 10 ml of acetic acid, and to the solution was added, at room temperature, 1.01 ml of bromine, and the mixture stirred at 90 °C for 5 hours.

After acetic acid was removed under a reduced pressure from the reaction mixture, a saturated aqueous sodium hydrogen carbonate was added until the solution became about pH 9, and the desired product was extracted from the mixture with ethyl acetate. The organic layer obtained was washed with saturated aqueous sodium chloride and dried over anhydrous magnesium sulfate, and the solution was concentrated under a reduced pressure. The solid obtained was purified by column chromatography to give about 1.8 g (ca. 50%) of 2-(α-bromoacetyl)pyrazine.

$^{1}$H-NMR (90 MHz, δ ppm, $CDCl_3$);

4.89 (s, 2H) 8.77 (d, J = 7.0 Hz, 2H)

9.10 (s, 1H)

A 207 mg amount of 6,7-dihydroxy-2-mercaptonaphthalene was dissolved in 15 ml of methylene chloride, and to the solution was added, at room temperature, a solution of 482 mg of 2-(α-bromoacetyl pyrazine obtained above in methylene chloride (3 ml), and the mixture was stirred for 3 hours.

To the reaction mixture was added saturated aqueous sodium hydrogen carbonate, and the desired product was extracted from the mixture with ethyl acetate. The organic layer obtained was washed with saturated aqueous sodium chloride and dried over anhydrous magnesium sulfate, and the solution was concentrated under a reduced pressure. After methylene chloride was added to the concentrate, insolubles in methylene chloride were removed by filtration, and the filtrate was again concentrated under a reduced pressure. The concentrate was purified by column chromatography to give 139 mg (46%) of (6,7-dihydroxy-2-naphthylthio)methyl 2-pyrazinyl ketone.

$^{1}$H-NMR (90 MHz, δ ppm, $d_6$-Acetone);

4.53 (s, 2H) 7.15 (d, J = 3.7 Hz, 2H)

7.2 - 8.7 (m, 6H)

Example 13

Synthesis of (6,7-dihydroxy-2-naphthylthio)methyl-2-pyrrolyl ketone

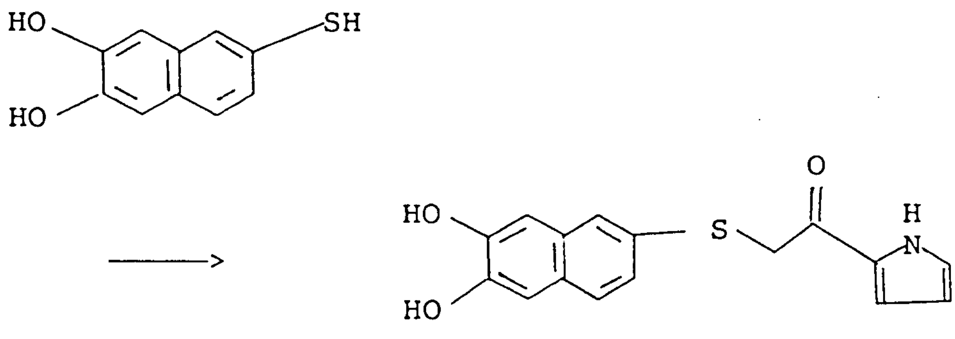

III-(6)

To a solution of 210 mg (1.09 mmol) of 6,7-dihydroxy-2-mercaptonaphthalene in 3 ml of pyridine and 5 ml of methylene chloride was added a 2 ml methylene chloride solution of 205 mg (1.09 mmol) of α-bromo-2-acetylpyrrole synthesized from bromine and 2-acetylpyrrole, and the mixture was stirred at room temperature for 5 hours. The reaction was completed with an aqueous potassium hydrogen sulfate, the mixture was extracted with ethyl acetate, and the extract was washed with water and saturated aqueous sodium chloride, and dried over anhydrous magnesium sulfate. After evaporation of the solvent, the residue was subjected to silica gel column chromatography to give 150 mg (46%) of (6,7-dihydroxy-2-naphthylthio)-methyl 2-pyrrolyl ketone.

$^1$H-NMR (90 MHz, δ ppm, $CDCl_3$);

4.55 (s, 2H) 6.26 (m, 1H) 6.85 - 7.60 (m, 7H)

Example 14

Synthesis of (6,7-dihydroxy-2-naphthylthio)methyl-2-thiazolyl ketone

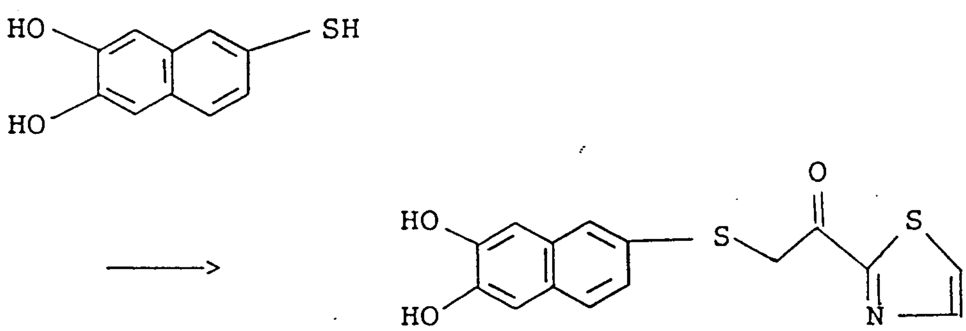

III-(7)

To a solution of 151 mg (0.79 mmol) of 6,7-dihydroxy-2-mercaptonaphthalene in 2 ml of pyridine was added a 2 ml methylene chloride solution of 163 mg (0.79 mmol) of α-bromo-2-acetyl-thiazole synthesized from bromine and 2-acetylthiazole, and the mixture was stirred at room temperature for 5 hours. The reaction was completed with an aqueous potassium hydrogen sulfate, the mixture was extracted with ethyl acetate, and the extract was washed with water and saturated aqueous sodium chloride, and dried over anhydrous magnesium sulfate. After evaporation of the solvent, the residue was subjected to silica gel column chromatography to give 144 mg (58%) of 6,7-dihydroxy-2-naphthylthio)methyl 2-thiazolyl ketone.

$^1$H-NMR (90 MHz, δ ppm, $CDCl_3$);

4.48 (s, 2H) 7.13 - 8.00 (m, 7H)

Example 15

Synthesis of (6,7-dihydroxy-2-naphthylthio)methyl-2-thienyl ketone

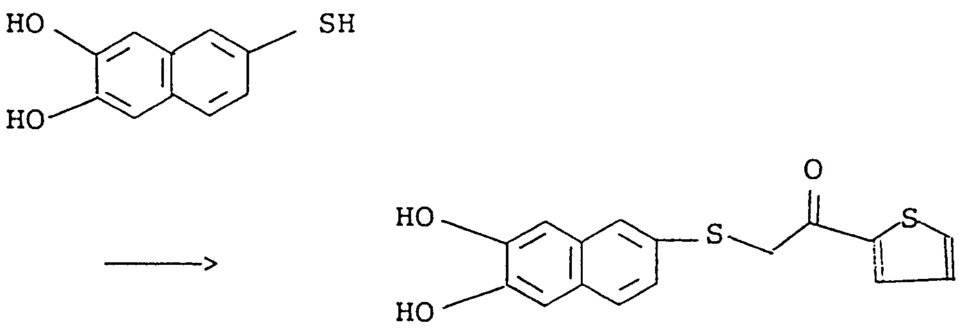

III-(8)

To a solution of 192 mg (1 mmol) of 6,7-dihydroxy-2-mercaptonaphthalene in 3 ml of pyridine was added a 2 ml methylene chloride solution of 206 mg (1 mmol) of α-bromo-2-acetyl-thiophen synthesized from bromine and 2-acetylthiophen, and the mixture stirred at room temperature for 4 hours. The reaction was completed with an aqueous potassium hydrogen sulfate, the mixture was extracted with ethyl acetate, and the extract was washed with water and saturated aqueous sodium chloride, and dried over anhydrous magnesium sulfate. After evaporation of the solvent, the residue was subjected to silica gel column chromatography to give 232 mg (73%) of (6,7-dihydroxy-2-naphthylthio)methyl 2-thiazolyl ketone.

$^1$H-NMR (90 MHz, δ ppm, $CDCl_3$);

4.50 (s, 2H) 7.05 - 7.90 (m, 8H)

Example 16

Synthesis of methyl (6,7-dihydroxy-2-naphthylthio)acetate (1)

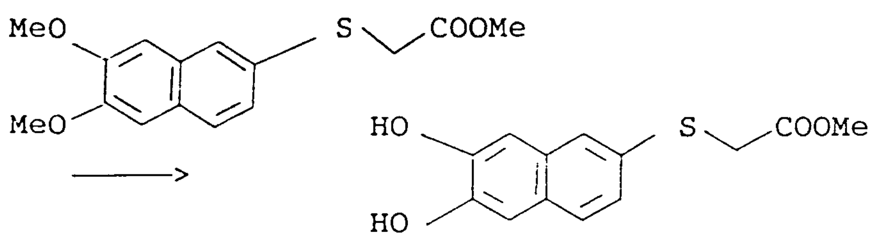

IV-(1)

To 2 ml of a dry methylene chloride solution of 174 mg (0.6 mmol) of the dimethoxy derivative (Example 32) was added, under $N_2$ atmosphere, 56 µl of $BBr_3$ at -78°C, and the mixture was stirred overnight while gradually increasing the temperature to room temperature. Water was added to the reaction system, and the mixture was extracted with ethyl acetate. After drying the extract, the solvent was evaporated under a reduced pressure, and subsequently, the oily product obtained was formed into a solution in 5 ml $CHCl_3$ and 1 ml MeOH, followed by an addition of 2 drops of $H_2SO_4$. The mixture was stirred at room temperature for 12 hours, water was added, the mixture was extracted with ethyl acetate, and the extract was washed three times with aqueous sodium chloride, dried, and the solvent was then evaporated. The oily product obtained was subjected to silica gel column chromatography (hexane:ethyl acetate = 2:1) to give 74 mg (47%) of the demethoxylated derivative.

$^1$H-NMR (90 MHz) (δ ppm, $CDCl_3$);

3.67 (s, 2H) 3.70 (s, 3H) 7.0 - 7.7 (m, 5H)

Example 17

Synthesis of methyl (6,7-dihydroxy-2-naphthylthio)acetate (2)

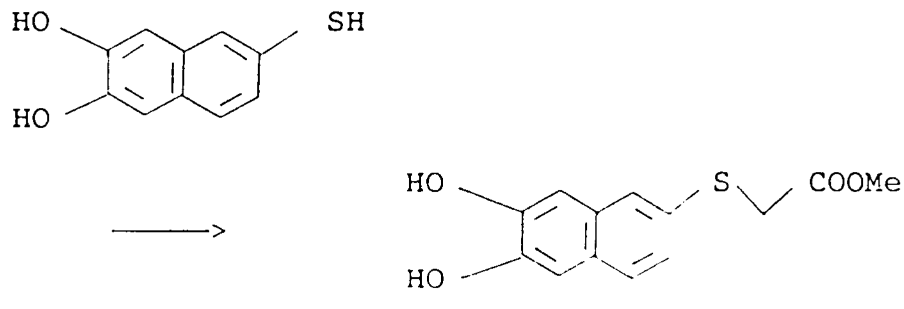

IV-(1)

A 50 ml pyridine solution of 2.55 g (13.3 mmol) of 6,7-dihydroxy-2-mercaptonaphthalene was cooled to 0 ° C, and 1.50 ml (2.44 g, 16.0 mmol) of methyl bromoacetate was added thereto, followed by stirring at room temperature for 3 hours. The reaction was completed by an addition of ether and saturated aqueous potassium hydrogen sulfate to the reaction system, and the mixture was extracted with ether. The organic layer was washed with saturated aqueous potassium hydrogen sulfate and with saturated aqueous sodium chloride, dried over anhydrous magnesium sulfate, and then filtered. The solvent was evaporated under a reduced pressure, and the residue was crystallized from chloroform to give 2.1 g (60%) of colorless crystals.

mp; 110 - 112 ° C

$^1$H-NMR (90 MHz, $\delta$ ppm, $CDCl_3$);

3.67 (s, 2H) 3.70 (s, 3H) 7.0 - 7.7 (m, 5H)

Example 18

Synthesis of (6,7-dihydroxy-2-naphthylthio)acetic acid

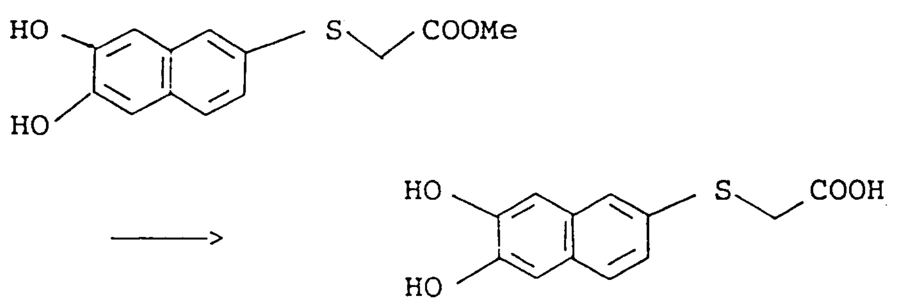

IV-(2)

To a solution of 331 mg (1.25 mmol) of methyl (6,7-dihydroxy-2-naphthylthio)acetate in methanol (2 ml) and THF (4 ml) was added 3 ml of a 4N aqueous LiOH solution, and the mixture was stirred at room temperature for 19 hours. The reaction was acidified with conc. hydrochloric acid, and extracted with ethyl acetate. The organic layer was washed with saturated aqueous sodium chloride, and then dried over anhydrous magnesium sulfate, and after evaporation of the solvent under a reduced pressure, the residue was subjected to Florisil column (hexane:ethyl acetate = 1:4) to give 262 mg (84%) of (6,7-dihydroxy-2-naphthylthio)acetic acid.

$^1$H-NMR (90 MHz, $\delta$ ppm, $CDCl_3$);

3.67 (s, 3H) 7.0 - 7.8 (m, 5H)

Example 19

Synthesis of 2-amino-2-hydroxymethyl-1,3-propane diol salt of (6,7-dihydroxy-2-naphthylthio)acetic acid

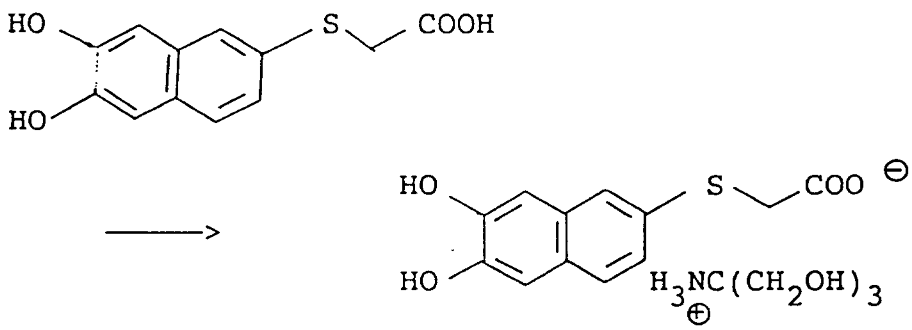

IV-(15)

Toga 2 ml methanolic solution of 197 mg of (6,7-dihydroxy-2-naphthylthio)acetic acid was added a 2 ml aqueous solution of 95.5 mg (0.79 mmol) of 2-amino-2-hydroxymethyl-1,3-propanediol, and after stirring for 1 hour, methanol was evaporated under a reduced pressure and the residual aqueous solution was freeze dried. Crystals in an amount of 290 mg (quant.) were obtained.

$^{1}$H-NMR (δ ppm, $D_2O$, TSP);

3.7 (s, 8H) 4.8 (s, 8H) 6.96 - 7.5 (m, 5H)

Example 20

Synthesis of butyl (6,7-dihydroxy-2-naphthylthio)acetate

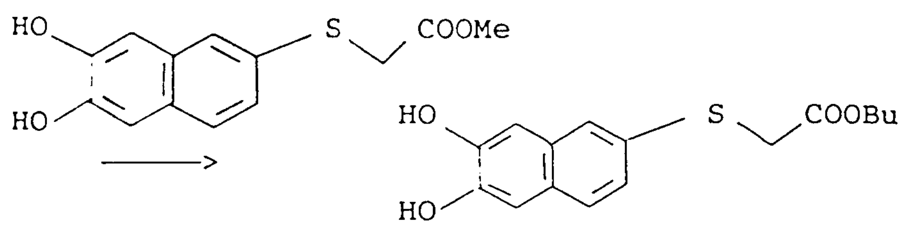

IV-(7)

A 224 mg amount of methyl (6,7-dihydroxy-2-naphthylthio)acetate was dissolved in 50 ml of butyl alcohol, to the solution was added 20 mg of p-toluenesulfonic acid, and the mixture was stirred at 100 °C for 12 hours. Further, the mixture was stirred for 3 hours while gradually distilling off the butyl alcohol.

Butyl alcohol was removed from the reaction mixture under a reduced pressure, the solution was neutralized with saturated aqueous sodium hydrogen carbonate, and the desired product was extracted from the mixture with ethyl acetate. The organic layer obtained was washed with saturated aqueous sodium chloride and dried over anhydrous magnesium sulfate, the solution was concentrated under a reduced pressure, and the concentrate was crystallized from ether and chloroform to give 193 mg (80%) of butyl (6,7-dihydroxy-2-naphthylthio)acetate.

$^{1}$H-NMR (90 MHz, δ ppm, $CDCl_3$);

0.94 (t, J = 4.2 Hz, 3H) 1.1 - 1.4 (m, 4H)

3.67 (s, 2H) 4.11 (t, J = 6.4 Hz, 2H)

5.99 (s, 2H) 7.09 (d, J = 2.9 Hz, 2H)

7.3 - 7.7 (m, 3H)

Example 21

Synthesis of tert-butyl (6,7-dihydroxy-2-naphthylthio)acetate

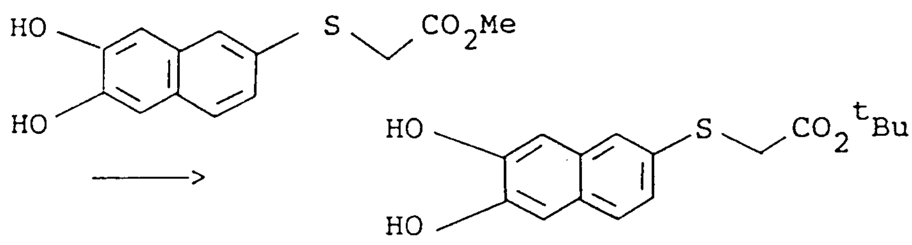

IV-(7)

A 229 mg amount of methyl (6,7-dihydroxy-2-naphthylthio)acetate was dissolved in 50 ml of tert-butyl alcohol, to the solution was added 20 mg of p-toluenesulfonic acid, and the mixture was stirred at 80 ° C for 15 hours. Further, the mixture was stirred for 3 hours while gradually distilling off the tert-butyl alcohol.

The tert-butyl alcohol was removed from the reaction mixture under a reduced pressure, the solution was neutralized with saturated aqueous sodium hydrogen carbonate, and the desired product was extracted from the mixture with ethyl acetate. The organic layer obtained was washed with saturated aqueous sodium chloride and dried over anhydrous magnesium sulfate, the solution was concentrated under a reduced pressure, and the concentrate was crystallized from ether and chloroform to give 90 mg (34%) of tert-butyl (6,7-dihydroxy-2-naphthylthio)acetate.

$^1$H-NMR (90 MHz, δ ppm, $CDCl_3$);

1.38 (s, 9H) 3.68 (s, 2H)

5.98 (s, 2H) 7.09 (d, J = 2.9 Hz, 2H)

7.3 - 7.7 (m, 3H)

Example 22

Synthesis of benzyl (6,7-dihydroxy-2-naphthylthio)acetate

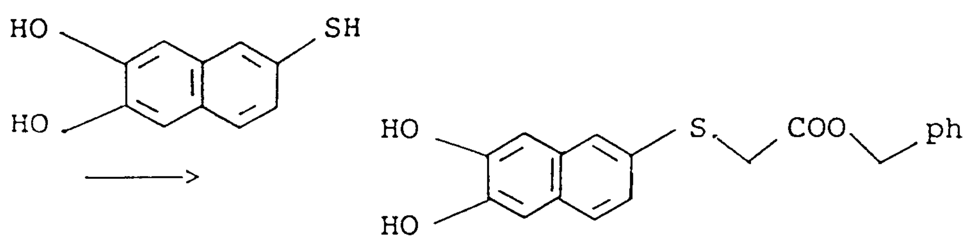

IV-(9)

Benzyl alcohol (1.03 ml) and triethylamine (1.67 ml) were dissolved in 30 ml of dichloromethane, to the solution was added 0.82 ml of bromoacetyl chloride at 0 ° C, and the mixture was stirred at 0 ° C for 4 hours.

The reaction mixture was filtered with Celite, and the insolubles were removed, followed by a neutralization of the solution with saturated aqueous potassium hydrogen sulfate. The desired product was extracted from the mixture with dichloromethane, the organic layer obtained was washed with saturated aqueous sodium chloride and dried over anhydrous magnesium sulfate, and the solution was concentrated under a reduced pressure. An amount of 1.98 g of the crude product of benzyl bromoacetate was obtained, and the product was used as such for the subsequent reaction.

$^1$H-NMR (90 MHz, δ ppm, $CDCl_3$);

3.86 (s, 2H) 5.20 (s, 2H) 7.36 (s, 5H)

A 312 mg amount of 6,7-dihydroxy-2-mercaptonaphthalene was dissolved in 20 ml of pyridine, to the

solution was added 1.24 g of the benzyl bromoacetate obtained above at 0 °C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture. The organic layer obtained was washed with saturated aqueous sodium chloride and dried over anhydrous magnesium sulfate, and the solution was concentrated under a reduced pressure. The concentrate was purified by column chromatography to give 405 mg (73%) of benzyl (6,7-dihydroxy-2-naphthylthio)acetate.

$^1$H-NMR (90 MHz, δ ppm, $CDCl_3$);
3.71 (s, 2H) 5.13 (s, 2H)
5.80 (s, 2H) 7.0 - 7.7 (m, 10H)

Example 23

Synthesis of methallyl (6,7-dihydroxy-2-naphthylthio)acetate

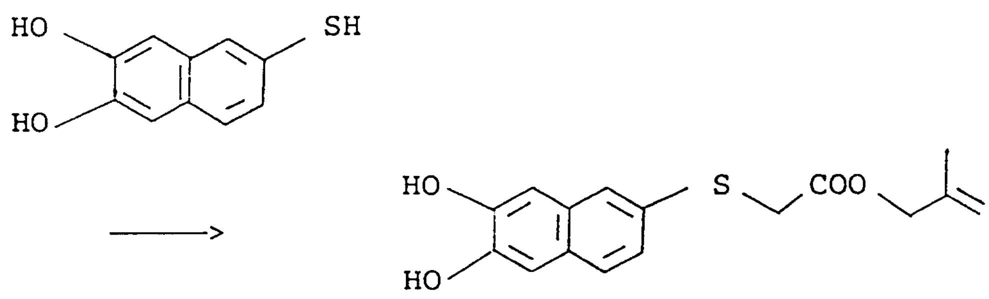

IV-(10)

Methallyl alcohol (0.84 ml) and triethylamine (1.67 ml) were dissolved in 30 ml of dichloromethane, to the solution was added 0.82 ml of bromoacetyl chloride at 0 °C, and the mixture was stirred at 0 °C for 4 hours.

The reaction mixture was filtered with Celite, and the insolubles were removed, followed by a neutralization of the solution with saturated aqueous potassium hydrogen sulfate. The desired product was extracted from the mixture with dichloromethane, and the organic layer obtained was washed with saturated aqueous sodium chloride and dried over anhydrous magnesium sulfate. The solution was concentrated under a reduced pressure, and an amount of 1.03 g of the crude product of methallyl bromoacetate was obtained. The product was used as such for the subsequent reaction.

$^1$H-NMR (90 MHz, δ ppm, $CDCl_3$);
1.73 (s, 3H) 3.87 (s, 2H) 4.17 (s, 2H)
4.87 (m, 2H)

An amount 318 mg of 6,7-dihydroxy-2-mercaptonaphthlene was dissolved in 20 ml of pyridine, to the solution was added 0.94 g of the methallyl bromoacetate obtained above at 0 °C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture. The organic layer obtained was washed with saturated aqueous sodium chloride and dried over anhydrous magnesium sulfate, and the solution was concentrated under a reduced pressure. The concentrate was purified by column chromatography to give 408 mg (81%) of methallyl (6,7-dihydroxy-2-naphthylthio)acetate.

$^1$H-NMR (90 MHz, δ ppm, $CDCl_3$);
1.73 (s, 3H) 3.76 (s, 2H) 3.89 (s, 1H)
4.87 (m, 2H) 5.63 (s, 2H)
7.1 - 7.8 (m, 5H)

Example 24

Synthesis of cyclohexyl (6,7-dihydroxy-2-naphthylthio)acetate

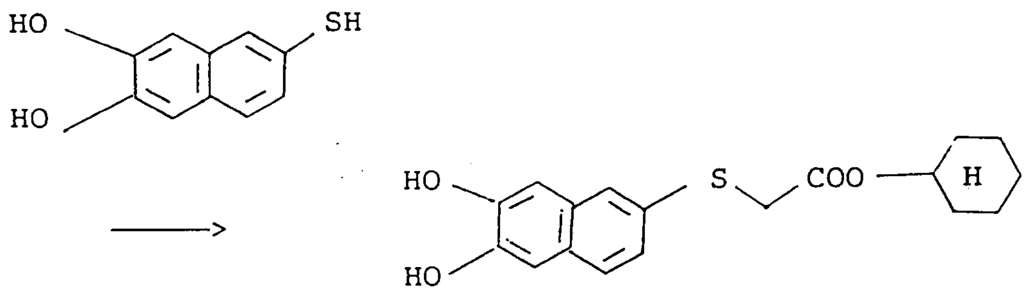

IV-(11)

Cyclohexanol (1.04 ml) and triethylamine (1.67 ml) were dissolved in 30 ml of dichloromethane, to the solution was added 0.82 ml of bromoacetyl chloride at 0 °C, and the mixture was stirred at 0 °C for 4 hours.

The reaction mixture was filtered with Celite, and the insolubles were removed, followed by a neutralization of the solution with saturated aqueous potassium hydrogen sulfate. The desired product was extracted from the mixture with dichloromethane, and the organic layer obtained was washed with saturated aqueous sodium chloride and dried over magnesium sulfate. The solution was concentrated under a reduced pressure, and an amount of 1.98 g of the crude product of cyclohexyl bromoacetate was obtained. The product was used as such for the subsequent reaction.

$^1$H-NMR (90 MHz, $\delta$ ppm, $CDCl_3$);

1.1 - 2.1 (m, 10H) 3.84 (s, 2H) 3.92 (m, 1H)

A 309 mg amount of 6,7-dihydroxy-2-mercaptonaphthalene was dissolved in 20 ml of pyridine, to the solution was added 1.04 g of the cyclohexyl bromoacetate obtained above at 0 °C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture. The organic layer obtained was washed with saturated aqueous sodium chloride and dried over anhydrous magnesium sulfate, and the solution was concentrated under a reduced pressure. The concentrate was purified by column chromatography to give 406 mg (76%) of cyclohexyl (6,7-dihydroxy-2-naphthylthio)acetate.

$^1$H-NMR (90 MHz, $\delta$ ppm, $CDCl_3$);

1.1 - 2.1 (m, 10H) 3.72 (s, 2H)

3.82 (m, 1H) 5.63 (s, 2H)

7.1 - 7.8 (m, 5H)

Example 25

Synthesis of (3,7-dimethyl)-6-octenyl (6,7-dihydroxy-2-naphthylthio)acetate

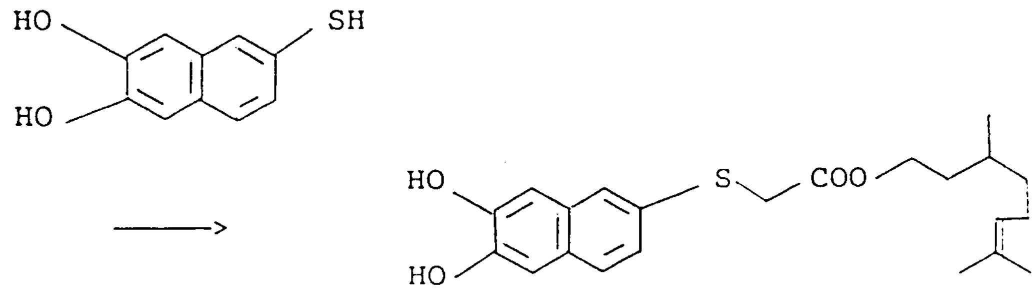

IV-(13)

Citronelol (1.82 ml) and triethylamine (1.67 ml) were dissolved in 30 ml of dichloromethane, to the solution was added 0.82 ml of bromoacetyl chloride at 0 ° C, and the mixture was stirred at 0 ° C for 4 hours.

The reaction mixture was filtered with Celite, and the insolubles were removed, followed by a neutralization of the solution with saturated aqueous potassium hydrogen sulfate. The desired product was extracted from the mixture with dichloromethane, and the organic layer obtained was washed with saturated aqueous sodium chloride and dried over anhydrous magnesium sulfate. The solution was concentrated under a reduced pressure, and an amount of 2.47 g of the crude product of (3,7-dimethyl)-6-octenyl bromoacetate was obtained. The product was used as such for the subsequent reaction.

$^1$H-NMR (90 MHz, δ ppm, $CDCl_3$);

0.92 (d, J = 5.1 Hz, 3 H) 1.1 - 2.1 (m, 7H)

1.60 (s, 3H) 1.69 (s, 3H)

3.81 (s, 2H) 4.22 (t, J = 6.6 Hz, 2H)

5.08 (t, J = 5.7 Hz, 1H)

A 306 mg amount of 6,7-dihydroxy-2-mercaptonaphthalene was dissolved in 20 ml of pyridine, to the solution was added 1.47 g of the (3,7-dimethyl)-6-octenyl bromoacetate obtained above at 0 ° C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture. The organic layer obtained was washed with saturated aqueous sodium chloride and dried over anhydrous magnesium sulfate, and the solution was concentrated under reduced pressure. The concentrate was purified by column chromatography to give 431 mg (70%) of (3,7-dimethyl)-6-octenyl (6,7-dihydroxy-2-naphthylthio)acetate.

$^1$H-NMR (90 MHz, δ ppm, $CDCl_3$);

0.84 (d, J = 5.7 Hz, 3H) 1.2 - 2.1 (m, 7H)

1.54 (s, 3H) 1.59 (s, 3H)

3.67 (s, 2H) 4.13 (t, J = 6.6 Hz, 2H)

5.08 (t, J = 5.7 Hz, 1H) 5.64 (s, 2H)

7.11 (d, J = 3.3 Hz 2H) 7.3 - 7.7 (m, 3H)

Example 26

Synthesis of (5-phenyl)pentyl (6,7-dihydroxy-2-naphthylthio)acetate

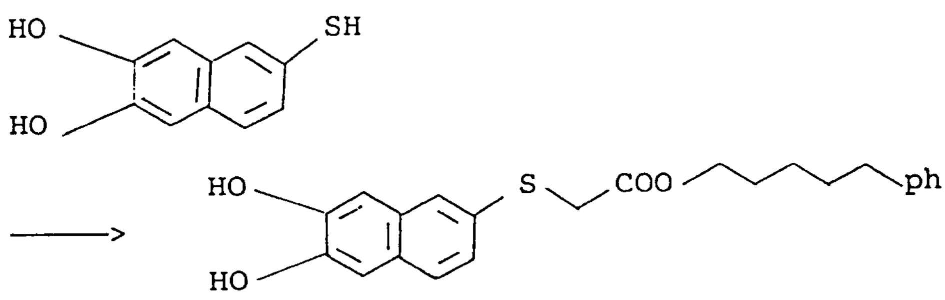

IV-(14)

In 30 ml of dichloromethane were dissolved 5-phenyl-1-pentanol (1.68 ml) and triethylamine (1:67 ml), to the solution was added 0.82 ml of bromoacetyl chloride at 0 °C, and the mixture was stirred at 0 °C for 4 hours.

The reaction mixture was filtered with Celite, and the insolubles were removed, followed by a neutralization of the solution with saturated aqueous potassium hydrogen sulfate. The desired product was extracted from the mixture with dichloromethane, and the organic layer obtained was washed with saturated aqueous sodium chloride and dried over anhydrous magnesium sulfate. The solution was concentrated under a reduced pressure, and an amount of 2.53 g of the crude product of (5-phenyl)pentyl bromoacetate was obtained. The product was used as such for the subsequent reaction.

$^1$H-NMR (90 MHz, δ ppm, $CDCl_3$);

1.1 - 2.1 (m, 6H) 2.62 (t, J = 7.7 Hz, 2H)

3.82 (s, 2H) 4.16 (t, J = 6.6 Hz, 2H)

7.1 - 7.3 (m, 5H)

A 313 mg amount of 6,7-dihydroxy-2-mercaptonaphthalene was dissolved in 20 ml of pyridine, to the solution was added 1.53 g of the (5-phenyl)pentyl bromoacetate obtained above at 0 °C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture. The organic layer obtained was washed with saturated aqueous sodium chloride and dried over anhydrous magnesium sulfate, and the solution was concentrated under a reduced pressure. The concentrate was purified by column chromatography to give 457 mg (71%) of (5-phenyl)pentyl (6,7-dihydroxy-2-naphthylthio)acetate.

$^1$H-NMR (90 MHz, δ ppm, $CDCl_3$);

1.2 - 2.2 (m, 6H) 2.62 (t, J = 7.7 Hz, 2H)

3.70 (s, 2H) 4.07 (t, J = 6.6 Hz, 2H)

5.64 (s, 2H) 7.1 - 7.7 (m, 10H)

Example 27

Synthesis of N-5-tetrazolyl(6,7-dihydroxy-2-naphthylthio)acetamide

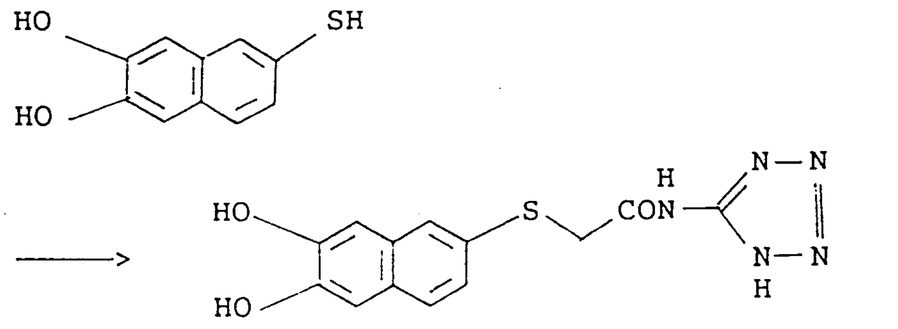

V-(2)

In 30 ml of dichloromethane were dissolved 5-aminotetrazole monohydrate (1.03 g) and triethylamine (2.1 ml), to the solution was added 1.25 ml of bromoacetyl chloride at 0 ° C, and the mixture was stirred at room temperature for 15 hours.

After saturated potassium hydrogen sulfate was added to the reaction mixture, dichloromethane was evaporated under a reduced pressure, and the desired product was extracted from the liquid obtained with ethyl acetate. The organic layer obtained was washed with saturated aqueous sodium chloride and dried over anhydrous magnesium sulfate, and the solution was concentrated under a reduced pressure. The solid obtained was recrystallized from ethyl acetate to give 767 mg (37%) of 5-(bromoacetylamino)tetrazole.

$^1$H-NMR (90 MHz, $\delta$ ppm, $d_6$-DMSO);

4.17 (s), 4.40 (s) ... total 2H

12.43 (br. s, 1H)

Then 6,7-dihydroxy-2-mercaptonaphthalene (769 mg) and 5-(bromoacetylamino)tetrazole (754 mg) were dissolved in 12 ml of pyridine, and the solution was stirred at room temperature for 20 hours.

The reaction mixture was ice-cooled, neutralized with 6N hydrochloric acid, and the desired product was extracted from the mixture with ethyl acetate. The organic layer obtained was washed with saturated aqueous sodium chloride and dried over anhydrous magnesium sulfate, and the solution was concentrated under a reduced pressure. The crystals obtained were washed with chloroform and ethyl acetate to give 692 mg of N-5-tetrazolyl(6,7-dihydroxy-2-naphthylthio)acetamide (52%, m.p. 227 - 229 ° C)

$^1$H-NMR (90 MHz, $\delta$ ppm, $d_6$-Acetone];

4.00 (s, 2H) 7.1 - 7.9 (m, 5H)

8.1 - 8.9 (m, 3H) 11.26 (br. s, 1H)

11.94 (br. s, 1H)

Example 28

Synthesis of N-(2-carboxyphenyl)(6,7-dihydroxy-2-naphthylthio)acetamide

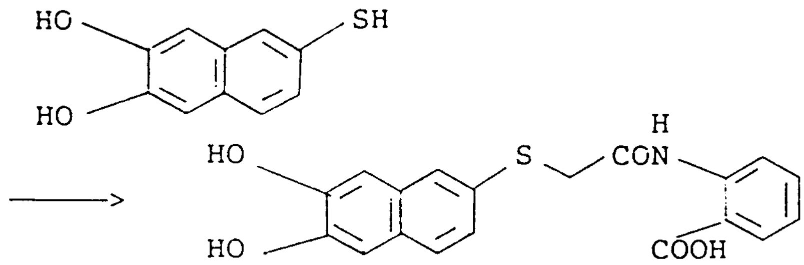

V-(3)

Anthranylic acid (547 mg) and triethylamine (0.7 ml) were dissolved in 30 ml of dichloromethane, to the solution was added 0.4 ml of bromoacetyl chloride at 0 °C, and the mixture was stirred at room temperature for 12 hours.

After saturated potassium hydrogen sulfate was added to the reaction mixture, dichloromethane was evaporated under a reduced pressure, and the desired product was extracted from the liquid obtained with ether. The organic layer obtained was washed with saturated aqueous sodium chloride and dried over anhydrous magnesium sulfate, and the solution was concentrated under a reduced pressure to give 1.02 g (99%) of 2-(bromoacetylamino)benzoic acid.

$^1$H-NMR (90 MHz, δ ppm, $CDCl_3$);
4.15 (s, 1H) 4.32 (s, 1H)
7.20 (dd, J = 7.5 & 7.5 Hz, 1H)
7.62 (ddd, J = 2.1, 7.5 & 7.5 Hz, 1H)
8.13 (dd, J = 2.1 & 7.5 Hz, 1H)
8.66 (d, J = 7.5 Hz),
8.70 (d, J = 7.5 Hz) .... total 1H
11.95 (br. s, 1H)

A 481 mg amount of 6,7-dihydroxy-2-mercaptonaphthalene and 968 mg of 2-(bromoacetylamino)-benzoic acid were dissolved in 10 ml of pyridine, and the solution was stirred at room temperature for 6.5 hours.

The reaction mixture was neutralized with saturated aqueous potassium hydrogensulfate, and the desired product was extracted from the mixture with ethyl acetate. The organic layer obtained was washed with saturated aqueous sodium chloride and dried over anhydrous magnesium sulfate, and the solution was concentrated under a reduced pressure. The concentrate was crystallized from acetone to give 859 mg of N-(2-carboxylphenyl)(6,7-dihydroxy-2-naphthylthio)acetamide (93%).

$^1$H-NMR (90 MHz, δ ppm, $d_6$-Acetone);
3.96 (s, 2H) 6.9 - 8.1 (m, 8H)
8.3 - 8.7 (m, 3H) 9.50 (br. s, 1H)
11.94 (br. s, 1H)

Example 29

Synthesis of N-(2-methoxycarbonylphenyl)(6,7-dihydroxy-2-naphthylthio)acetamide

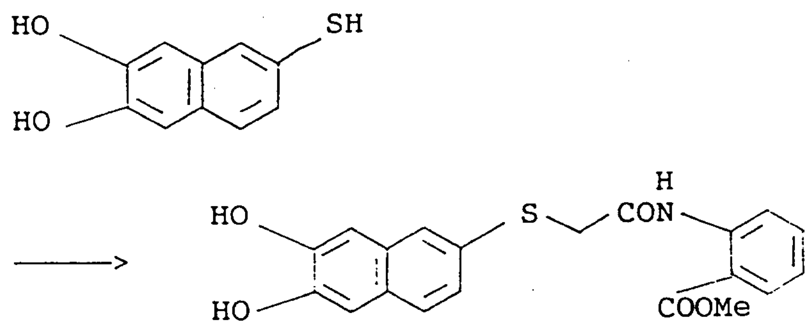

V-(4)

Methyl anthranylate (153 mg) and triethylamine (0.17 ml) were dissolved in 3 ml of dichloromethane, to the solution was added 185 mg of bromoacetyl chloride at room temperature, and the mixture was stirred at room temperature for 2 hours.

After saturated sodium hydrogen sulfate was added to the reaction mixture, dichloromethane was evaporated under a reduced pressure, and the desired product was extracted from the liquid obtained with ether. The organic layer obtained was washed with saturated aqueous sodium chloride and dried over anhydrous magnesium sulfate, and the solution was concentrated under a reduced pressure. The concentrate was purified by column chromatography to give 244 mg (93%) of methyl 2-(bromoacetylamino)-benzoate.

$^1$H-NMR (90 MHz, $\delta$ ppm, $CDCl_3$);
3.95 (s, 3H) 4.02 (s, 1H)
4.20 (s, 1H) 7.13 (dd, J = 7.5 & 7.5 Hz, 1H)
7.56 (ddd, J = 1.8, 7.5 & 7.5 Hz, 1H)
8.05 (dd, J = 1.8 & 7.5 Hz, 1H)
8.66 (d, J = 7.5 Hz, 1H)

A 203 mg amount of 6,7-dihydroxy-2-mercaptonaphthalene and 206 mg of methyl 2-(bromoacetylamino)benzoate were dissolved in 4 ml of pyridine, and the solution was stirred at room temperature for 4 hours.

The reaction mixture was neutralized with saturated aqueous sodium chloride, and the desired product was extracted from the mixture with ethyl acetate. The organic layer obtained was washed with saturated aqueous sodium chloride and dried over anhydrous magnesium sulfate, and the solution was concentrated under a reduced pressure. The concentrate was purified by column chromatography to give 195 mg (67%) of N-(2-methoxycarbonylphenyl)6,7-dihydroxy-2-naphthylthio)acetamide.

$^1$H-NMR (90 MHz, $\delta$ ppm, $CDCl_3$);
3.91 (s, 3H) 3.92 (s, 2H)
7.0 - 7.6 (m, 7H)
7.99 (dd, J = 1.8 & 7.5 Hz, 1H)
8.45 (br, s, 2H) 8.65 (d, J = 8.5 Hz, 1H)

Example 30

Synthesis of N-[2-(5-tetrazolyl)phenyl](6,7-dihydroxy-2-naphthylthio)acetamide

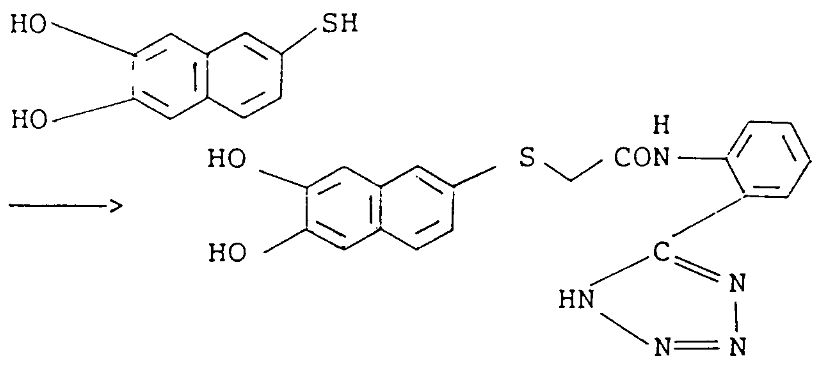

V-(5)

Anthranilonitrile (1.79 g) and triethylamine (2.5 ml) were dissolved in 45 ml of dichloromethane, to the solution was added 1.5 ml of bromoacetyl chloride at 0 °C, and the mixture was stirred at room temperature for 12 hours.

After saturated potassium hydrogen sulfate was added to the reaction mixture, dichloromethane was evaporated under a reduced pressure, and the desired product was extracted from the liquid obtained with ether and ethyl acetate. The organic layer obtained was washed with saturated aqueous sodium chloride and dried over anhydrous magnesium sulfate, and the solution was concentrated under a reduced pressure. The liquid obtained was purified by column chromatography to give 1.79 g (49%) of N-(2-cyanophenyl)-bromoacetamide.

$^1$H-NMR (90 MHz, δ ppm, $CDCl_3$);
4.07 (s), 4.24 (s) ... total 2H
7.1 - 7.4 (m, 1H) 7.4 - 7.8 (m, 2H)
8.37 (d, J = 9.0 Hz, 1H) 8.5 - 9.0 (br. 1H)

A 1.19 g amount of 6,7-dihydroxy-2-mercaptonaphthalene and 1.78 g of N-(2-cyanophenyl)-bromoacetamide were dissolved in 25 ml of pyridine, and the solution was stirred at room temperature for 12 hours.

The reaction mixture was ice-cooled, and then neutralized with 6N hydrochloric acid, and the desired product was extracted from the mixture with ethyl acetate. The organic layer obtained was washed with saturated aqueous sodium chloride and dried over anhydrous magnesium sulfate, and the solution was concentrated under a reduced pressure. The liquid obtained was crystallized from chloroform to give 1.80 g (83%) of N-(2-cyanophenyl)(6,7-dihydroxy-2-naphthylthio)acetamide.

$^1$H-NMR (90 MHz, δ ppm, $d_6$-Acetone);
3.99 (s, 2H) 7.0 - 8.2 (m, 9H)
8.3 - 8.8 (br., 2H) 9.3 - 9.6 (br., 1H)

A 646 mg amount of N-(2-cyanophenyl)(6,7-dihydroxy-2-naphthylthio)acetamide, 649 mg of sodium azide, and 646 mg of ammonium chloride were dissolved in 4 ml of dimethylformamide, and the solution was stirred at 120 °C for 16 hours.

The reaction mixture was cooled to room temperature, and then neutralized with 2N hydrochloric acid, and the desired product was extracted from the mixture with ethyl acetate. The organic layer obtained was washed with saturated aqueous sodium chloride and dried over anhydrous magnesium sulfate, and the solution was concentrated under a reduced pressure. The liquid obtained was purified by column chromatography to give 139 mg (95%) of N-[2-(5-tetrazolyl)phenyl](6,7-dihydroxy-2-naphthylthio)acetamide.

$^1$H-NMR (90 MHz, δ ppm, $d_6$-Acetone);
4.02 (s, 2H) 6.9 - 7.8 (m, 8H)
7.9 - 8.1 (m, 1H) 8.67 (s, 1H)
8.75 (s, 1H) 11.60 (br. s, 1H)

Example 31

Synthesis of 1-[4-(6,7-dihydroxy-2-naphthylthio)acetylamino)butyl]-4-[bis(4-fluorophenyl)methyl]piperazine

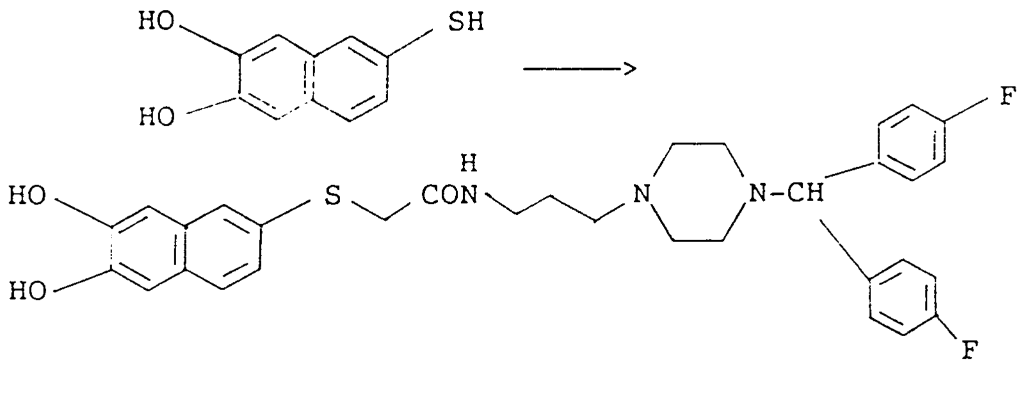

V-(6)

To a solution of 16.2 g of 1,4-bromobutane dissolved in 40 ml of dimethylformamide was added, at room temperature, 4.63 g of potassium phthalimide, and the mixture was stirred for 2 hours, and further the stirring was continued at 80 ° C for 30 minutes.

After cooling to room temperature, water was added to the reaction mixture, and the desired product was extracted therefrom with ether. The organic layer obtained was washed with saturated aqueous sodium chloride and dried over anhydrous magnesium sulfate, and the solution was concentrated under a reduced pressure. The concentrate was purified by column chromatography to give 6.13 g (87%) of N-4-(bromobutyl)phthalimide.

$^1$H-NMR (90 MHz, $\delta$ ppm, $CDCl_3$);
1.6 - 2.1 (m, 4H) 3.2 - 3.6 (m, 2H)
3.5 - 3.9 (m, 2H) 7.5 - 8.0 (m, 4H)

N-(4-bromobutyl)phthalimide (2.27 g), 1-[bis(4-fluorophenyl)methyl]piperazine (2.31 g), potassium carbonate (1.66 g) and sodium iodide (1.87 g) were dissolved in 40 ml of 2-butanone, and the solution was refluxed for 6 hours.

After cooling to room temperature, the reaction mixture was filtered to remove insolubles, the insolubles were washed with chloroform, and the filtrate and the washing were combined and concentrated under a reduced pressure. The liquid obtained was dissolved in chloroform, washed with water, and dried over anhydrous magnesium sulfate. The solution was concentrated under a reduced pressure, and the concentrate was purified by column chromatography to give 3.79 g (96%) of 1-(4-succinimidobutyl)-4-[bis(4-fluorophenyl)methyl]piperazine.

$^1$H-NMR (90 MHz, $\delta$ ppm, $CDCl_3$);
1.2 - 2.0 (m, 4H) 2.0 - 2.7 (m, 2H)
3.69 (t, J = 6.6 Hz, 2H) 4.19 (s, 1H)
6.7 - 7.1 (m, 4H) 7.1 - 7.5 (m, 4H)
7.6 - 8.0 (m, 4H)

Then 1-(4-succinimidobutyl)-4-[bis(4-fluorophenyl)methyl]piperazine (3.76 g) and saturated aqueous hydrazine (0.794 g) were dissolved in 25 ml of ethanol, and the solution was refluxed for 2 hours.

After cooling to room temperature, the reaction mixture was filtered to remove insolubles, the filtrate obtained was concentrated under a reduced pressure, and chloroform was added to the liquid obtained. The insolubles in the chloroform were filtered with Celite, the filtrate obtained was washed with water and dried over magnesium sulfate, and the solution was concentrated under a reduced pressure to give 2.44 g (93%) of 1-(4-aminobutyl)-4-[bis(4-fluorophenyl)methyl]piperazine.

$^1$H-NMR (90 MHz, $\delta$ ppm, $CDCl_3$);
1.1 - 1.9 (m, 4H) 2.2 - 3.1 (m, 14H)
4.22 (s, 1H) 6.7 - 7.2 (m, 4H)
7.1 - 7.3 (m, 4H)

Then 1-(4-aminobutyl)-4-[bis(4-fluorophenyl)methyl]piperazine (2.42 g) was dissolved in 22 ml of dichloromethane, to the solution was added 0.88 ml of bromoacetyl chloride at 0°C, and the mixture was stirred at room temperature.

After water was added to the reaction mixture, dichloromethane was removed under a reduced pressure, and to the liquid obtained was added saturated aqueous sodium hydrogen carbonate, to adjust the pH to 9. The desired product was extracted from the mixture with ethyl acetate, the organic layer obtained was washed with saturated aqueous sodium chloride and dried over anhydrous magnesium sulfate, and the solution was concentrated under a reduced pressure. The concentrate was purified by column chromatography to give 2.41 (74%) g of 1-[4-bromoacetylamino)butyl]-4-[bis(4-fluorophenyl)methyl]-piperazine.

$^1$H-NMR (90 MHz, δ ppm, $CDCl_3$);
1.4 - 2.3 (m, 4H) 2.5 - 3.6 (m, 12H)
3.89 (s), 4.02 (s) ... total 2H
4.37 (s, 1H) 6.6 - 7.7 (m, 8H)

Then 6,7-dihydroxy-2-mercaptonaphthalene (0.91 g) and 1-[4-(bromoacetylamino)butyl]-4-[bis(4-fluorophenyl)methyl]piperazine (2.40 g) were dissolved in 20 ml of pyridine, and the solution was stirred at room temperature for 40 hours.

The reaction mixture was neutralized with 6N hydrochloric acid, and the desired product was extracted from the mixture with ethyl acetate. The organic layer obtained was washed with saturated aqueous sodium hydrogen carbonate and saturated aqueous sodium chloride and dried over anhydrous magnesium sulfate, and the solution was concentrated under a reduced pressure. The concentrate was purified by column chromatography to give 0.40 g (14%) of 1-[4-(6,7-dihydroxy-2-naphthylthio)acetyl-amino)butyl]-4-[bis(4-fluorophenyl)methyl]piperazine.

$^1$H-NMR (90 MHz, δ ppm, $CDCl_3$);
1.1 - 1.7 (m, 4H) 1.9 - 2.9 (m, 10H)
2.9 - 3.4 (m, 2H) 3.59 (s, 2H)
4.27 (s, 1H) 6.7 - 7.6 (m, 13H)

Example 32

Synthesis of methyl (6,7-dimethoxy-2-naphthylthio)acetate

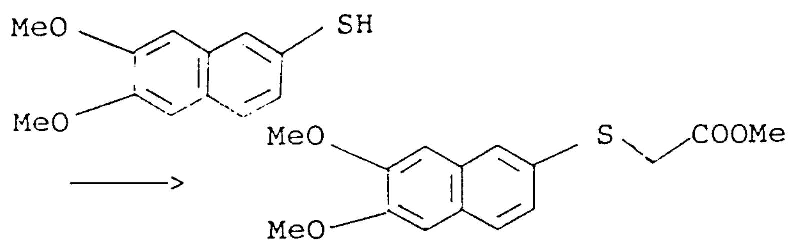

VI-(1)

To 10 ml of a dry DMF solution of 1 g (4.5 mmol) of 6,7-dimethoxy-2-mercaptonaphthalene was added 200 mg (5 mmol) of NaH (60% in oil) at 0°C, the mixture was added to 4 ml of a dry DMF solution of 474 ml (765 mg, 5 mmol) of methyl bromoacetate at 0°C, and the mixture was stirred at 0°C for 4 hours. To the reaction system was added saturated aqueous ammonium chloride, the mixture was extracted with ether, and the organic layer was dried over anhydrous magnesium sulfate and filtered, followed by evaporation of the solvent. The oily product thus obtained was subjected to silica gel column chromatography (hexane:ethyl acetate = 4:1) to give 1.04 g (78%) of methyl (6,7-dimethoxy-2-naphthylthio)acetate.

$^1$H-NMR (90 MHz, δ ppm, $CDCl_3$);
3.8 (s, 5H) 4.0 (s, 6H) 7.0 (s like, 2H)
7.33 (dd, 1H, J = 10.0, 2.5 Hz)
7.60 (d, 1H, J = 10.0 Hz) 7.73 (s like, 1H)
$^1$H-NMR (90 MHz, δ ppm, $C_6D_6$)
3.3 (s, 3H) 3.46 (s, 8H) 6.8 (s, 2H)
7.45 (s, 1H) 7.46 (s, 1H) 7.84 (s, 1H)

Example 33

Synthesis of methyl (6,7-diacetoxy-2-naphthylthio)acetate

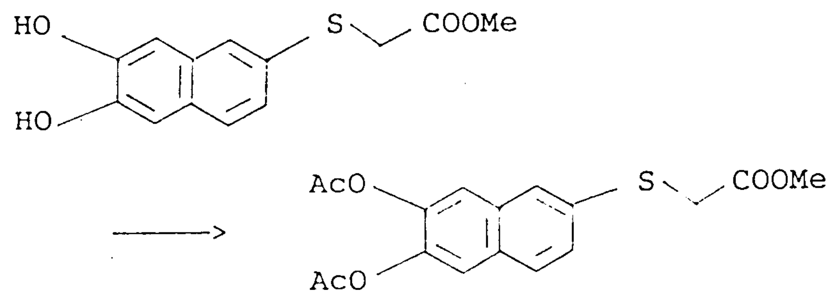

VI-(2)

A solution of 135 mg (0.51 mmol) of methyl (6,7-dihydroxy-2-naphthylthio)acetate in pyridine (2 ml) was cooled to 0 °C, and acetyl chloride (80 μl, 1.1 mmol) was added, followed by stirring for one hour, and further, at room temperature for 4 hours. The reaction was completed by an addition of an aqueous potassium hydrogen sulfate, the mixture was extracted with ether, and the organic layer was washed with saturated aqueous sodium chloride and dried over anhydrous magnesium sulfate. The solvent was evaporated under a reduced pressure, and the oily product thus obtained was subjected to silica gel chromatography (hexane:ethyl acetate = 5:1 → 3:1) to obtain 156 mg (87%) of methyl (6,7-diacetoxy-2-naphthylthio)acetate.

$^1$H-NMR (90 MHz, δ ppm, $CDCl_3$);
2.33 (s, 6H) 3.70 (s, 3H)
3.73 (s, 2H) 7.3 - 7.83 (m, 5H)

Example 34

Synthesis of methyl (6,7-dimethoxycarbonyloxy-2-naphthylthio)acetate

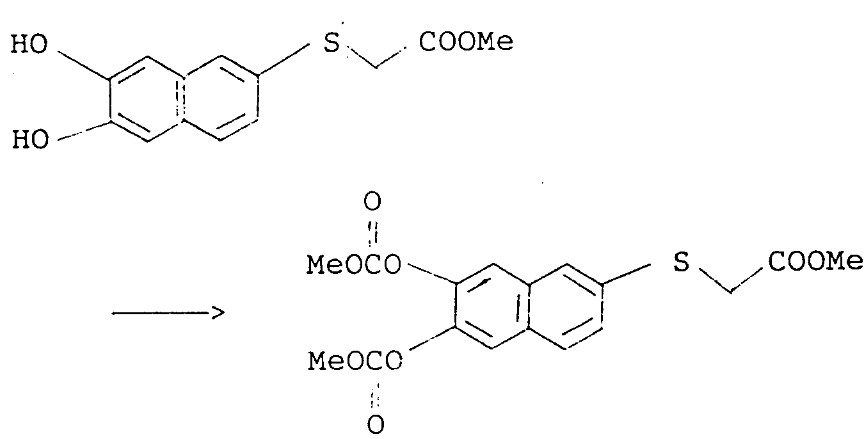

VI-(3)

A solution of 175 mg (0.66 mmol) of methyl (6,7-dihydroxy-2-naphthylthio)acetate in pyridine (2 ml) was cooled to 0 °C, and methyl chloroformate (123 μl, 1.46 mmol) was added, followed by stirring for one hour, and further, at room temperature for 4 hours. The reaction was completed by an addition of an aqueous potassium hydrogen sulfate, the mixture was extracted with ether, and the organic layer was washed with saturated aqueous sodium chloride and dried over anhydrous magnesium sulfate. The solvent was evaporated under a reduced pressure, and the oily product thus obtained was subjected to silica gel

chromatography (hexane:ethyl acetate = 5:1 → 3:1) to obtain 203 mg (80%) of methyl (6,7-dimethoxycarbonyloxy-2-naphthylthio)acetate.

$^1$H-NMR (90 MHz, δ ppm, $CDCl_3$);

3.70 (s, 5H) 3.93 (s, 6H) 7.2 - 7.85 (m, 5H)

Example 35

Synthesis of methyl (6,7-diisopropoxycarbonyloxy-2-naphthylthio)acetate

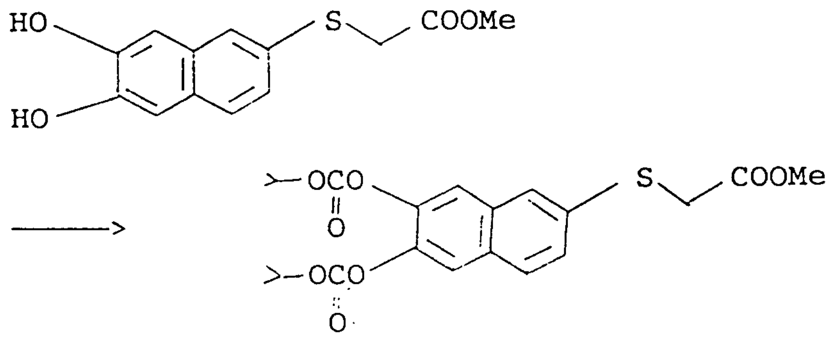

VI-(4)

To a solution of 216 mg (0.82 mmol) of methyl 6,7-dihydroxy-2-naphthylthioacetate in pyridine (2 ml) was added 204 μl (1.80 mmol) of isopropyl chloroformate, and the mixture was stirred for one hour.

The reaction was completed by an addition of ether and saturated aqueous potassium hydrogen sulfate, the mixture was extracted with ether, and the organic layer was washed with saturated aqueous potassium chloride and saturated aqueous sodium chloride and dried over anhydrous magnesium sulfate, followed by filtration. The solvent was evaporated under a reduced pressure to obtain 349 mg (98%) of colorless crystals.

mp; 75 - 77 °C

$^1$H-NMR (90 MHz, δ ppm, $CDCl_3$);

1.38 (d, 12H J = 7.0 Hz) 3.71 (s, 3H)

3.73 (s, 2H) 4.96 (q, 1H, J = 7.0 Hz)

5.03 (q, 1H, J = 7.0 Hz) 7.4 - 7.8 (m, 5H)

Example 36

Synthesis of methyl [6,7-bis(dimethylcarbamoyloxy)-2-naphthylthio]acetate

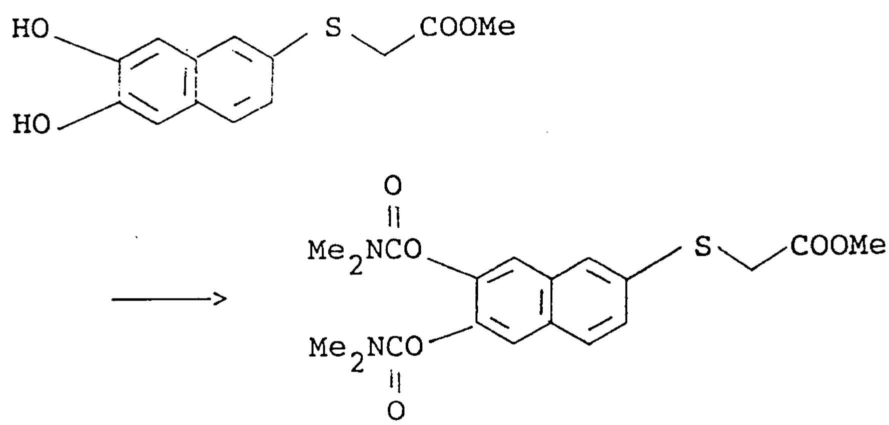

VI-(5)

To a solution of 223 mg of methyl (6,7-dihydroxy-2-naphthylthio)acetatein 1 ml of pyridine was added 0.32 ml of dimethylcarbamyl chloride at room temperature, followed by stirring for 5.5 hours.

To the reaction mixture was added 3N hydrochloric acid to neutralize the pyridine, the product was extracted with ethyl acetate, and the organic layer obtained was washed with saturated aqueous ammonium chloride and saturated aqueous sodium chloride. The solution was dried over anhydrous magnesium sulfate, and the solvent then concentrated under a reduced pressure. The concentrate was purified by column chromatography to obtain 222 mg (64%) of methyl [6,7-bis(dimethylcarbamoyloxy)-2-naphthylthio)acetate.

$^1$H-NMR (90 MHz, δ ppm, $CDCl_3$);

3.04 (s, 6H) 3.10 (s, 6H)

3.70 (s, 5H) 7.42 (dd, J = 1.8 & 8.6 Hz, 1H)

7.5 - 7.8 (m, 4H)

Example 37

Synthesis of methyl 2-(6,7-dihydroxy-2-naphthylthio)propionate

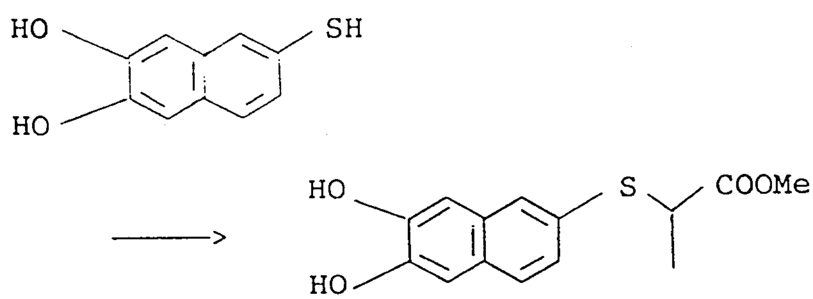

VII-(1)

A 1.00 g amount of 6,7-dihydroxy-2-mercaptonaphthalene was dissolved in 50 ml of pyridine, to the solution was added dℓ methyl 2-bromopropionate at 0 ° C, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture with ether. The organic layer obtained was washed with saturated aqueous sodium chloride and dried over anhydrous magnesium sulfate, and the solution was concentrated under a reduced pressure. The desired

product was separated by column chromatography, and crystallized from ether and chloroform to give 952 mg of methyl 2-(6,7-dihydroxy-2-naphthylthio)propionate (66%, m.p. 112 - 114 ° C).

$^1$H-NMR (90 MHz, $\delta$ ppm, $CDCl_3$);
1.50 (d, J = 7.0 Hz, 3H)
3.66 (s, 3H) 3.83 (q, J = 7.0 Hz, 1H)
5.64 (s, 1H) 5.67 (s, 1H)
7.1 - 7.8 (m, 5H)

Example 38

Synthesis of methyl 2-methyl-2-(6,7-dihydroxy-2-naphthylthio)propionate

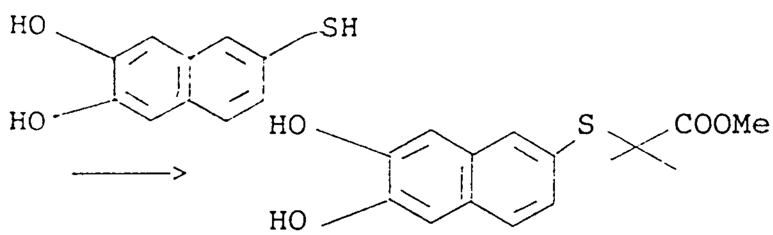

VII-(2)

To a 10 ml pyridine solution of 240 mg (1.25 mmol) of 6,7-dihydroxy-2-mercaptonaphthalene was added a 2 ml methylene chloride solution of 209 mg (1.25 mmol) of methyl 2-bromo-2-methyl-propionate, and the mixture was stirred at room temperature for 12 hours. The reaction was completed with an aqueous magnesium hydrogen sulfate, the mixture was extracted with ethyl acetate, and the extract was washed with water and saturated aqueous sodium chloride and dried over anhydrous magnesium sulfate. After evaporation of the solvent under a reduced pressure, the residue was subjected to silica gel column chromatography to give 222 mg (64%) of methyl 2-methyl-2-(6,7-dihydroxy-2-naphthylthio)propionate.

$^1$H-NMR (90 MHz, $\delta$ ppm, $CDCl_3$);
1.70 (s, 6H) 3.65 (s, 3H) 7.1 - 7.8 (m, 5H)

Example 39

Synthesis of methyl 2-(6,7-dihydroxy-2-naphthylthio)phenylacetate

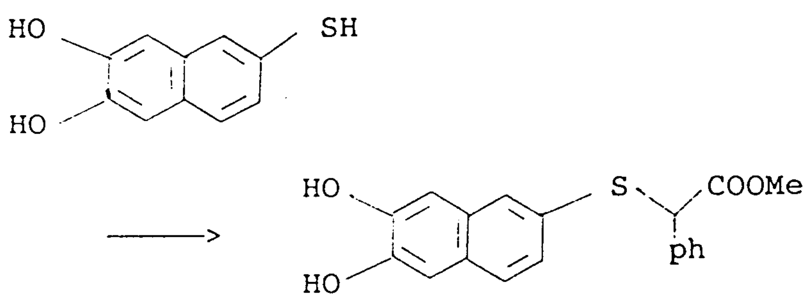

VII-(3)

To a 10 ml pyridine solution of 241 mg (1.25 mmol) of 6,7-dihydroxy-2-mercaptonaphthalene was added a 3 ml methylene chloride solution of 286 mg (1.25 mmol) of methyl $\alpha$-bromophenylacetate, and the mixture was stirred at room temperature for 24 hours. The reaction was completed with an aqueous potassium hydrogen sulfate, the mixture was extracted with ethyl acetate, and the extract was washed with water and saturated aqueous sodium chloride, and dried over anhydrous magnesium sulfate. After evaporation of the solvent under a reduced pressure, the residue was subjected to silica gel column

chromatography to give 208 mg (49%) of methyl 2-(6,7-dihydroxy-2-naphthylthio)phenylacetate.

$^1$H-NMR (90 MHz, $\delta$ ppm, $CDCl_3$);

3.65 (s, 3H) 4.85 (s, 1H) 7.10 - 7.70 (m, 10H)

Example 40

Synthesis of 2-(benzylsulfonyl)-6,7-dihydroxynaphthalene

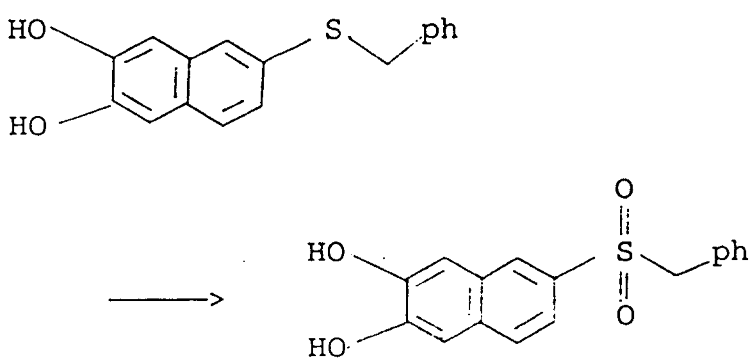

VIII-(1)

A 16 mg amount of 2-(benzylthio)-6,7-dihydroxynaphthalene was dissolved in 2 ml of dichloromethane, to the solution was added 45 mg of m-chlorobenzoic acid at room temperature, and the mixture was stirred for 12 hours.

The reaction mixture was neutralized with saturated aqueous sodium hydrogen carbonate, and the desired product was extracted from the mixture with ethyl acetate. The organic layer obtained was washed with saturated aqueous sodium chloride and dried over magnesium sulfate, the solution was concentrated under a reduced pressure, and the concentrate was purified by column chromatography to give 10 mg (58%) of 2-(benzylsulfonyl)-6,7-dihydroxynaphthalene.

$^1$H-NMR (90 MHz, $\delta$ ppm, $d_6$-Acetone);

4.44 (s, 2H) 7.2 - 8.1 (m, 10H)

Example 41

Synthesis of 2-(benzylsulfinyl)-6,7-dihydroxynaphthalene

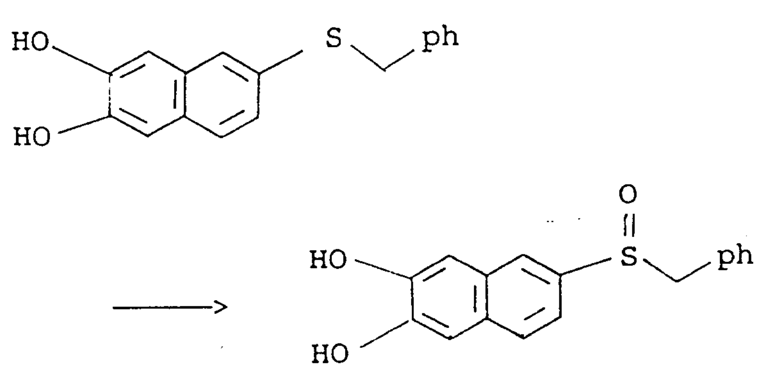

VIII-(2)

A 13 mg amount of 2-(benzylthio)-6,7-dihydroxynaphthalene was dissolved in 1 ml of acetone, to the solution was added 95 $\mu$l of 30% aqueous hydrogen peroxide, and the mixture was stirred for 12 hours.

To the reaction mixture was added saturated aqueous sodium sulfite and ethyl acetate, to separate the mixture into two layers. The aqueous layer was extracted with ethyl acetate, and the organic layer was washed with saturated aqueous sodium chloride and dried over anhydrous magnesium sulfate. The solution was concentrated under a reduced pressure, and the concentrate was purified by column chromatography to give 11 mg (79%) of 2-(benzylsulfinyl)-6,7-dihydroxynaphthalene.

$^1$H-NMR (90 MHz, δ ppm, $d_6$-Acetone);
4.11 (s), 4.14 (s) ... total 2H
7.1 - 7.9 (m, 10H)

Example 42

Synthesis of methyl (6,7-dihydroxy-2-naphthylsulfinyl)acetate

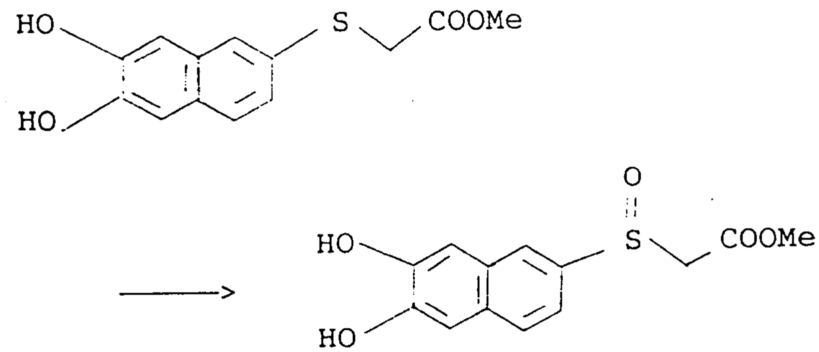

VIII-(3)

A 264 mg amount of methyl (6,7-dihydroxy-2-naphthylthio)acetate was dissolved in 1 ml of acetone, to the solution was added 0.12 ml of 30% aqueous hydrogen peroxide at room temperature, and the mixture was stirred for 12 hours.

To the reaction mixture were added saturated aqueous sodium sulfite and ethyl acetate, to separate the mixture into two layers. The aqueous layer was extracted with ethyl acetate, and the organic layer obtained was washed with saturated aqueous sodium chloride and dried over anhydrous magnesium sulfate. The solution was concentrated under a reduced pressure, and the concentrate was purified by column chromatography to give 176 mg (63%) of methyl (6,7-dihydroxy-2-naphthylsulfinyl)acetate.

$^1$H-NMR (90 MHz, δ ppm, $d_6$-DMSO);
3.62 (s, 3H) 3.91 (d, J = 14.1 Hz, 1H)
4.11 (d, J = 14.1 Hz, 1H) 7.20 (s, 1H)
7.25 (s, 1H)
7.43 (dd, J = 8.6 & 1.8 Hz, 1H)
7.76 (d, J = 8.6 Hz, 1H)
7.93 (d, J = 1.8 Hz, 1H)

Example 43

Synthesis of (6,7-dihydroxy-2-naphthylthio)acetonitrile

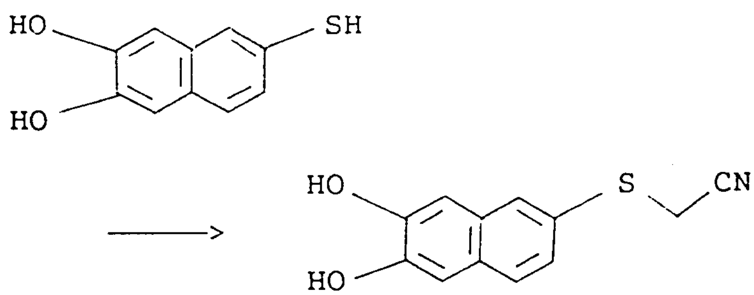

IX-(1)

A 1.00 g amount of 6,7-dihydroxy-2-mercaptonaphthalene was dissolved in 50 ml of pyridine, to the solution was added 0.72 ml of bromoacetonitrile, and the mixture was stirred at room temperature for 12 hours.

Pyridine was removed from the reaction mixture under a reduced pressure, the solution was neutralized with saturated aqueous potassium hydrogen sulfate, and the desired product was extracted from the mixture with ether. The organic layer obtained was washed with saturated aqueous sodium chloride, and dried over anhydrous magnesium sulfate, the solution was concentrated under a reduced pressure, and the concentrate was crystallized from chloroform to give 794 mg (66%, m.p. 143 - 146°C) of 6,7-dihydroxy-2-naphthylthio)acetonitrile.

$^1$H-NMR (90 MHz, δ ppm, $d_6$-Acetone);

2.75 (s, 2H) 3.94 (s, 2H)

7.3 - 8.0 (m, 4H) 8.54 (s, 1H)

Example 44

IgE antibody production suppressive effect of synthesized compound

An 8 weeks old BALB/C mouse (♀) was immunized by an intraperitoneal injection of 10 μg of TNP-KLH (trinitrophenyl-key hole limpet hemocyanine) and 2 mg of aluminum hydroxide gel, and after 3 weeks, 1 μg of TNP-KLH and 2 mg of aluminum hydroxide gel were boosted, and 4 weeks later, the spleen was enucleated.

The spleen cells (6 x $10^6$) were cultured together with 10 ng/ml of TNP-KLH, in the presence or non-presence of the compound of the present invention ($10^{-5}$ to $10^{-7}$ M), in 1 ml of RPMI-1640 medium (containing 10% fetal bovine serum) for 2 days, followed by washing to remove the antigen and the drug, and the washed cells were resuspended in a fresh medium containing no antigen and further cultured for 5 days. All cultivations were performed under 5% $CO_2$ at 37°C. After completion of the culturing, the supernatant was recovered, and the concentration of anti-TNP IgE or anti-TNP IgG antibody was determined by an antigen and isotype specific immunoassay. As shown in Table 1, methyl 6,7-dihydroxy-2-naphthylthioacetate (Exemplary compound IV (1)) suppressed the anti-TNP IgE antibody production dose-dependency, but had substantially no suppressive effect against the production of anti-TNP IgG. When the antigen-dependent IgE production was compared with the value of the spontaneous IgE production, a suppressive ratio of 70 - 90% was observed in Compound IV-(1). Detailed data are shown in Table 1.

Table 1

| Effect of thionaphthalene derivative on IgE and IgG antibody production | | | | |
|---|---|---|---|---|
| Antigen TNP-KLH (10 ng/ml) | Drug added | | Anti-TMP antibody production | |
| | Compound | Concentration (M) | IgE (ng/ml) | IgG ($\mu$g/ml) |
| + | No addition | - | 13.0 (8.5) | 8.1 |
| + | Exemplary compound IV-(1) | $10^{-7}$ | 7.5 (2.5) | 7.5 |
| + | " | $10^{-6}$ | 5.5 (1.0) | 7.3 |
| - | No addition | - | 4.5 | 0.8 |

The values in the brackets ( ) indicate the values of the spontaneous IgE production amount observed when TNP-KLH was not added, and represent the IgE production dependency on the antigen.

The same experimental methods were used for the compounds shown below, and IgE antibody production suppressive effects were evaluated. Also, in this case, substantially no suppressive effect was exhibited against the production of IgG. The results are summarized in Table 2.

(Exemplary compound VI - (1)):
methyl (6,7-dimethoxy-2-naphthylthio)acetate

(Exemplary compound V - (4)):
N-(2-methoxycarbonylphenyl)-(6,7-dihydroxy-2-naphthylthio)acetamide

(Exemplary compound V - (2)):
N-tetrazolyl(6,7-dihydroxy-2-naphthylthio)acetamide

Table 2

| Effect of thionaphthalene derivative on IgE antibody production | | |
|---|---|---|
| Compound | Concentration | Suppressive ratio |
| VI-(1) | $10^{-6}$ M | 69% |
| V -(4) | $10^{-7}$ M | 26% |
| " | $10^{-6}$ M | 7% |
| V -(2) | $10^{-7}$ M | 11% |

Example 45

Histamine release from peritoneal mast cells of rat

To a Sprague-Dawley strain male rat (10 weeks or older) was intraperitoneally administered, under ether anesthesia, a diluted rat anti-DNP-As IgE serum at 2 ml/kg. After 16 hours, cells were recovered by washing intraperitoneally with a Tyrode solution containing 0.5% heparin ($Ca^{++}$, $Mg^{++}$, free). Mast cells (5 x $10^4$) separated by overlying in the 30% Ficoll/Tyrode solution and centrifugation were reliberated into the Tyrode solution ($Ca^{++}$, $Mg^{++}$) and incubated with the addition of the drug solution shown in Table 3-1, for 10 minutes, and then DNP-As and phosphatidylserine were added to final concentrations of 20 $\mu$g/ml and 25 $\mu$g/ml, respectively, followed by incubation for 10 minutes. The reaction was stopped by ice-cooling, and the histamine release in the supernatant was determined by fluorophotometry.

Regarding the non-allergy release, 5 x $10^4$ of mast cells were investigated by incubation with 0.5 $\mu$g/ml of Compound 48/80 for 10 minutes, after the addition of the drug solution shown in Table 3-2. The results are shown in Table 3-1 and 3-2.

Table 3-1

Inhibitory activities on histamine release (anti-DNP-IgE)

| Exemplary compound | Concentration (M) | Inhibitory ratio (%) |
|---|---|---|
| Experiment 1 | | |
| IV-(1) | $10^{-6}$ | 13.4 |
| " | $10^{-5}$ | 63.3 |
| Control compound | | |
| Tranilast | $10^{-5}$ | 35.7 |
| " | $10^{-4}$ | 43.2 |
| Experiment 2 | | |
| IV-(1) | $10^{-5}$ | 2.9 |
| IV-(2) | $10^{-5}$ | 59.3 |
| IV-(3) | $2 \times 10^{-5}$ | 50.8 |
| IV-(4) | $10^{-5}$ | 25.8 |
| Tranilast | $10^{-5}$ | 10.3 |
| Experiment 3 | | |
| IV-(1) | $10^{-5}$ | 58.0 |
| V-(3) | $10^{-5}$ | -0.3 |
| V-(2) | $10^{-5}$ | -8.1 |
| V-(5) | $10^{-5}$ | -6.1 |
| Tranilast | $10^{-5}$ | -8.4 |
| Experiment 4 | | |
| I-(7) | $10^{-5}$ | 24.5 |
| III-(1) | $10^{-5}$ | 10.8 |

Table 3-1 (continued)

Inhibitory activities on histamine release (anti-DNP-IgE)

| Exemplary compound | Concentration (M) | Inhibitory ratio (%) |
|---|---|---|
| Experiment 5 | | |
| IV-(1) | $10^{-5}$ | 67.8 |
| VII-(1) | $10^{-5}$ | 18.5 |
| IX-(1) | $10^{-5}$ | 4.3 |
| II-(6) | $10^{-5}$ | -1.8 |
| IV-(3) | $10^{-5}$ | 10.0 |
| Experiment 6 | | |
| IV-(1) | $10^{-5}$ | 65.9 |
| Control compound | | |
| Tranilast | $10^{-4}$ | 9.8 |
| Amlexanox | $10^{-4}$ | 19.8 |
| DSCG | $10^{-4}$ | 6.3 |

Table 3-2

| Inhibitory activities on histamine release (Compound 48/80; Non-allergic) | | |
|---|---|---|
| Exemplary compound | Concentration (M) | Inhibitory ratio (%) |
| Experiment 1 | | |
| IV-(1) | $10^{-6}$ | -1.9 |
| " | $10^{-5}$ | 10.5 |
| " | $10^{-4}$ | 23 |
| V-(6) | $10^{-5}$ | 5.9 |
| V-(5) | $10^{-5}$ | 28.1 |
| V-(3) | $10^{-5}$ | 1.3 |
| V-(4) | $10^{-5}$ | -0.8 |
| V-(2) | $10^{-5}$ | 2.2 |
| Tranilast | $10^{-5}$ | 24.4 |
| Experiment 2 | | |
| IV-(1) | $10^{-5}$ | 2 |
| VI-(1) | $10^{-5}$ | 3.7 |
| VI-(2) | $10^{-5}$ | 2.6 |
| VI-(3) | $10^{-5}$ | 7.5 |
| VI-(4) | $10^{-5}$ | 17.8 |

From the above results, it is seen that the compounds of the present invention, such as exemplary compounds IV-(1), IV-(2), and IV-(3), exhibit suppressive actions primarily against allergy histamine release and weak suppressive actions against non-allergy histamine release, and thus can be considered compounds having a selectivity.

Example 46

Rat homologous PCA reaction

The back of a Sprague-Dawley strain male rat (6 - 8 w) was depilated, and at one site on both sides thereof, 0.1 ml of a rat anti-ovalbumin IgE serum diluted with physiological saline was intradermally injected under ether anesthesia. After 48 hours, 25 mg/kg of ovalbumin, 25 mg/kg of Evans Blue were intravenously administered to induce the PCA reaction, and after 30 minutes, the skin was peeled off, dipped in a mixture of 0.3% $Na_2SO_4 \cdot 10H_2O$/Acetate (3:7), and the dye amount infiltrated was quantitated by absorbance at 620 nm.

The drug listed in Table 4 was suspended in 5% gum arabic solution and orally administered 1 hour before the antigen administration. The results are shown in Table 4.

Table 4

| Rat homologous PCA reaction | | | |
|---|---|---|---|
| Exemplary compound | Dose (mg/kg, p.o.) | Suppressive ratio (%) | |
| | | Experiment 1 | Experiment 2 |
| IV-(1) | 25 | 14.8 | 24.2 |
| IV-(1) | 50 | NT | 31.3 |
| V-(5) | 25 | NT | 16.5 |
| V-(6) | 25 | NT | 15.9 |
| V-(3) | 25 | -18.0 | NT |
| V-(4) | 25 | 0 | NT |
| V-(2) | 25 | -0.3 | NT |
| Control compound | | | |
| Tranilast | 200 | 24.2 | 26.9 |

Example 47

Lipoxygenase inhibitory activity evaluation

To 1 ml of heparin-treated vein blood of a healthy man without an administration of drugs was added 1 µl of a DMSO solution of the drug test sample listed in Table 5 (final $10^{-5}$ M), and after treatment at 37 °C for 5 minutes, 5 µl of a DMSO solution of A23187 was added (final 25 µM) and the treatment carried out at 37 °C for 15 minutes, followed by ice-cooling. After the addition of 10 µl of a DMSO solution of 100 ng of 15-HETE as the internal standard substance for quantitation, 0.8 ml of acetonitrile was added, and the precipitates formed were removed by centrifugation. Then, $LTB_4$ and 5-HETE in the supernatant were subjected to HPLC separation and quantitation (ref. F.J. Sweeney, et al., Prostaglandins Leukotrienes & Med., 28, 73 (1987)). The results are shown in Table 5.

Table 5

| Lipoxygenase inhibitory activity ($10^{-5}$ M) | | |
|---|---|---|
| Exemplary compound | Amount of $LTB_4$ produced (ng/ml/plasma) (Inhibitory ratio %) | Amount of 5-HETE produced (ng/ml/plasma) (Inhibitory ratio %) |
| VII-(1) | 35 (72) | 40 (69) |
| II-(6) | 39 (69) | 75 (43) |
| II-(1) | 30 (76) | 45 (66) |
| IV-(1) | 32 (74) | 52 (60) |
| No addition | 125 | 130 |

Example 48

Tablets having the following composition per one tablet were prepared.

| | |
|---|---|
| Active ingredient | 1 mg or 5 mg |
| Lactose | 280 mg |
| Potato starch | 80 mg |
| Polyvinyl pyrrolidone | 11 mg |
| Magnesium stearate | 5 mg |
| | 377 mg or 381 mg |

The active ingredient, lactose, and potato starch were mixed, and the mixture uniformly wetted with a 20% ethanolic solution of polyvinyl pyrrolidone, passed through a 20 mm mesh sieve, dried at 45 °C, and passed through a 15 mm mesh sieve. The thus-obtained granules were mixed with magnesium stearate and compressed to form tablets.

The exemplary compound (IV-(1)) was employed as a representative of the active ingredient.

Example 49

Hard gelatin capsules having the following composition per one capsule were prepared.

| | |
|---|---|
| Active ingredient | 1 mg or 5 mg |
| Microcrystalline cellulose | 195 mg |
| Amorphous silic acid | 5 mg |
| | 201 mg or 205 mg |

The active ingredient was finely pulverized, and the microcrystalline cellulose and unpressed amorphous silic acid were thoroughly mixed therewith, and then packed in hard gelatin capsules.

The exemplary compound (IV-(2)) was employed as representative of the active ingredient.

## Claims

**1.** A thionaphthalene derivative having the following formula [I], or a non-toxic salt thereof:

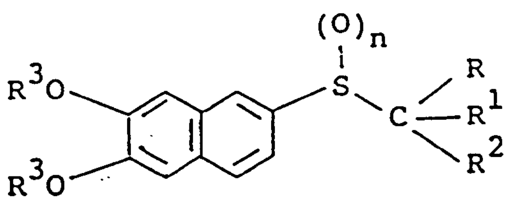

[I]

wherein R represents a hydrogen atom, an unsubstituted $C_1$ - $C_5$ alkyl group, a substituted or unsubstituted aryl group, a heterocyclic group, a group

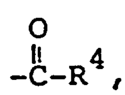

(where $R^4$ represents a substituted or unsubstituted $C_1$ - $C_4$ alkyl group, a substituted or unsubstituted aryl group or a heterocyclic group), a group

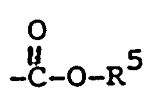

(where $R^5$ represents a hydrogen atom, a substituted or unsubstituted $C_1$ - $C_{10}$ alkyl group, a substituted or unsubstituted $C_3$ - $C_{10}$ alkenyl group or a substituted or unsubstituted $C_5$ - $C_7$ cycloalkyl group), a group

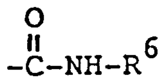

(where $R^6$ represents a substituted or unsubstituted $C_1$ - $C_4$ alkyl group; a substituted or unsubstituted aryl group or a heterocyclic group) or cyano group,

$R^1$ and $R^2$ each independently represent a hydrogen atom, a $C_1$ - $C_4$ alkyl group or a phenyl group,

$R^3$ represents a hydrogen atom, a $C_1$ - $C_4$ alkyl group or

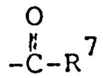

(where $R^7$ represents $-OR^{81}$, $-R^{82}$ or $-NR^{83}{}_2$ , and $R^{81}$, $R^{82}$, and $R^{83}$ each represent a $C_1$ - $C_4$ alkyl group), and n represents an integer of 0 to 2, with the proviso that R, $R^1$ and $R^2$ cannot all represent H when n is 0.

**2.** A thionaphthalene derivative or a non-toxic salt thereof as claimed in Claim 1 wherein, in the formula [I], R is a group

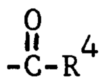

(where $R^4$ represents a $C_1$-$C_5$ alkyl group or a $C_6$ - $C_{10}$ aryl group), a group

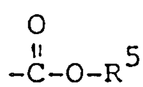

($R^5$ represents a hydrogen atom, a $C_1$ - $C_6$ alkyl group which may be substituted with a phenyl group, a $C_3$ - $C_{10}$ alkenyl group or a $C_5$ - $C_6$ cycloalkyl group).

**3.** A thionaphthalene derivative or a non-toxic salt thereof as claimed in claim 1 wherein, in the formula [I], $R^1$ and $R^2$ are each independently a hydrogen atom or a $C_1$ - $C_4$ alkyl group.

**4.** A thionaphthalene derivative or a non-toxic salt thereof as claimed in claim 1 wherein, in the formula [I], $R^3$ is a hydrogen atom or a group

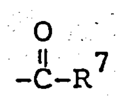

(where $R^7$ represents a $C_1$ - $C_4$ alkyl group).

**5.** A thionaphthalene derivative or a non-toxic salt thereof as claimed in claim 1 wherein, in the formula [I], n is 0.

**6.** A thionaphthalene derivative or a non-toxic salt thereof as claimed in claim 2 wherein, in the formula [I], $R^1$ and $R^2$ are each independently a hydrogen atom or a $C_1$ - $C_4$ alkyl group, and $R^3$ is a hydrogen atom or

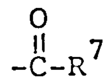

(where $R^7$ represents a $C_1$ - $C_4$ alkyl group).

**7.** A thionaphthalene derivative or a non-toxic salt thereof as claimed in claim 6 wherein, in the formula [I], R is

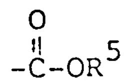

(where $R^5$ represents a hydrogen atom or a $C_1$ - $C_5$ alkyl group which may be substituted with phenyl group).

**8.** A method of preparing a thionaphthalene derivative having the formula [I']:

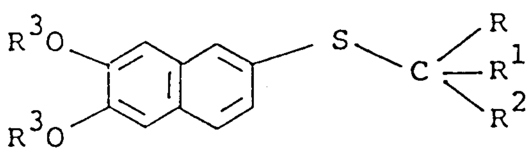

[I']

wherein R, $R^1$, $R^2$, and $R^3$ are as defined in Claim 1,which comprises reacting a thiol having the formula [II]:

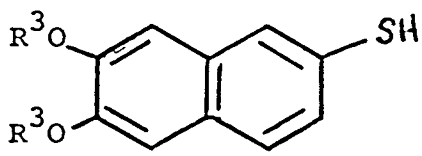

[II]

wherein $R^3$ is as defined in Claim 1 with a halide compound having the formula [III]:

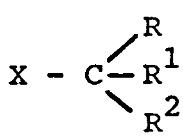

[III]

wherein X represents a halogen atom, R, $R^1$, $R^2$ are as defined in Claim 1, in the presence of a basic compound, with the proviso that R, $R^1$, and $R^2$ cannot all represent H.

**9.** A method of preparing a thionaphthalene derivative having the formula [I"]:

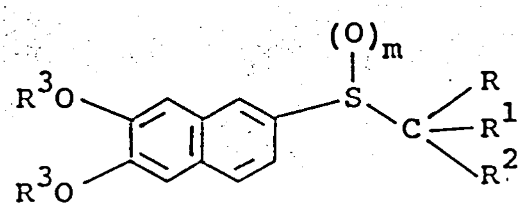

[I"]

wherein R, $R^1$, $R^2$, and $R^3$ are as defined in Claim 1 and m represents 1 or 2 which comprises reacting a thionaphthalene derivative having the formula [I']:

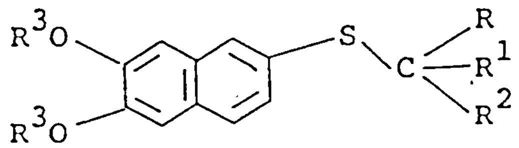

[I']

wherein R, $R^1$, $R^2$, and $R^3$ are as defined in Claim 1 with an oxidizing agent.

**10.** An antiallergy preparation comprising a thionaphthalene derivative having the above formula [I], or a non-toxic salt thereof, according to claim 1 as the active ingredient, together with a pharmaceutically acceptable carrier.

**Patentansprüche**

**1.** Thionaphthalinderivat der folgenden Formel (I) oder ein nicht-toxisches Salz hiervon:

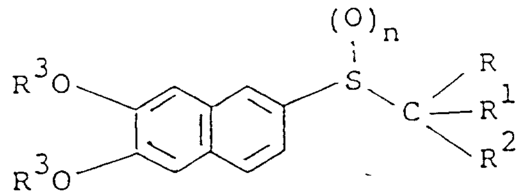

[I]

worin R ein Wasserstoffatom, eine unsubstituierte $C_1$ -$C_5$-Alkylgruppe, eine substituierte oder unsubstituierte Aryl-Gruppe, eine heterocyclische Gruppe,

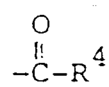

(in der $R^4$ eine substituierte oder unsubstituierte $C_1$ -$C_4$-Alkyl-Gruppe, eine substituierte oder unsubstituierte Aryl-Gruppe oder eine heterocyclische Gruppe repräsentiert), eine

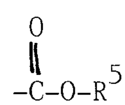

-Gruppe (in der $R^5$ ein Wasserstoffatom, eine substituierte oder unsubstituierte $C_1$ - $C_{10}$-Alkyl-Gruppe, eine substituierte oder unsubstituierte $C_3$ - $C_{10}$-Alkenyl-Gruppe oder eine substituierte oder unsubstituierte $C_5$ - $C_7$-Cycloalkyl-Gruppe repräsentiert), eine

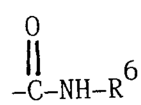

-Gruppe (worin $R^6$ eine substituierte oder unsubstituierte $C_1$ - $C_4$-Alkyl-Gruppe, eine substituierte oder unsubstituierte Aryl-Gruppe oder eine heterocyclische Gruppe repräsentiert) oder eine Cyano-Gruppe repräsentiert,
worin $R^1$ und $R^2$ jeweils unabhängig voneinander ein Wasserstoffatom, eine $C_1$ - $C_4$-Alkyl-Gruppe oder eine Phenyl-Gruppe repräsentiert,
worin $R^3$ ein Wasserstoffatom, eine $C_1$ - $C_4$-Alkyl-Gruppe oder

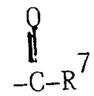

repräsentiert (worin $R^7$ $-OR^{81}$, $-R^{82}$ oder $NR^{83}{}_2$ repräsentiert und $R^{81}$, $R^{82}$ und $R^{83}$ jeweils eine $C_1$ - $C_4$-Alkyl-Gruppe repräsentieren)
und worin n eine ganze Zahl von 0 bis 2 repräsentiert, unter der Voraussetzung, daß R, $R^1$ und $R^2$ nicht alle ein Wasserstoffatom repräsentieren, wenn n gleich O ist.

**2.** Thionaphthalinderivat oder ein nicht-toxisches Salz hiervon nach Anspruch 1, worin in der Formel (I) R eine

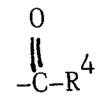

-Gruppe (worin $R^4$ eine $C_1$ - $C_5$-Alkyl-Gruppe oder eine $C_6$ - $C_{10}$-Aryl-Gruppe repräsentiert) oder eine

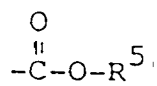

-Gruppe ist (worin $R^5$ ein Wasserstoffatom, eine $C_1$ - $C_6$-Alkyl-Gruppe, welche mit einer Phenyl-Gruppe substituiert sein kann, eine $C_3$ - $C_{10}$-Alkenyl-Gruppe oder eine $C_5$ - $C_6$-Cycloalkyl-Gruppe repräsentiert).

**3.** Thionaphthalinderivat oder ein nicht-toxisches Salz hiervon gemäß Anspruch 1, worin in der Formel (I) $R^1$ und $R^2$ jeweils unabhängig voneinander ein Wasserstoffatom oder eine $C_1$ - $C_4$-Alkyl-Gruppe sind.

**4.** Thionaphthalinderivat oder ein nicht-toxisches Salz hiervon gemäß Anspruch 1, worin in der Formel (I) $R^3$ ein Wasserstoffatom oder eine

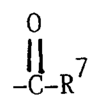

-Gruppe ist (worin $R^7$ eine $C_1$ - $C_4$-Alkyl-Gruppe repräsentiert).

**5.** Thionaphthalinderivat oder ein nicht-toxisches Salz hiervon gemäß Anspruch 1, worin in der Formel (I) n gleich Null ist.

**6.** Thionaphthalinderivat oder ein nicht-toxisches Salz hiervon gemäß Anspruch 2, worin in der Formel (I) $R^1$ und $R^2$ jeweils unabhängig voneinander ein Wasserstoffatom oder eine $C_1$ - $C_4$-Alkyl-Gruppe sind und $R^3$ ein Wasserstoffatom oder

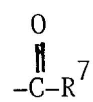

ist (worin $R^7$ eine $C_1$ -$C_4$-Alkyl-Gruppe repräsentiert).

**7.** Thionaphthalinderivat oder ein nicht-toxisches Salz hiervon gemäß Anspruch 6, worin in der Formel (I) R

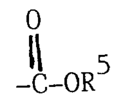

ist (worin $R^5$ ein Wasserstoffatom oder eine $C_1$ -$C_5$-Alkyl-Gruppe, welche mit einer Phenyl-Gruppe substituiert sein kann, repräsentiert).

**8.** Verfahren zur Herstellung eines Thionaphthalinderivats mit der Formel (I'):

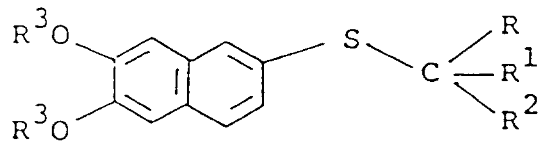

[I']

worin R, $R^1$, $R^2$ und $R^3$ wie in Anspruch 1 definiert sind, welches das Reagierenlassen eines Thiols der Formel (II):

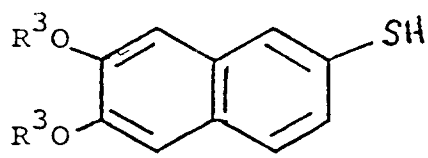

[II]

in der $R^3$ wie in Anspruch 1 definiert ist,
mit einer Halogenid-Verbindung der Formel (III) umfaßt:

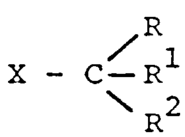

[III]

worin X ein Halogenatom repräsentiert, R, $R^1$ und $R^2$ wie in Anspruch 1 definiert sind,
in Gegenwart einer basischen Verbindung, mit der Bedingung, daß R, $R^1$ und $R^2$ nicht alle Wasserstoff repräsentieren können.

**9.** Verfahren zur Herstellung eines Thionaphthalinderivats der Formel (I"):

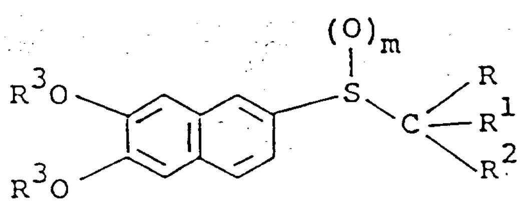

[I"]

worin R, $R^1$, $R^2$ und $R^3$ wie in Anspruch 1 definiert sind und m 1 oder 2 repräsentiert,
welches das Reagierenlassen eines Thionaphthalinderivats der Formel (I'):

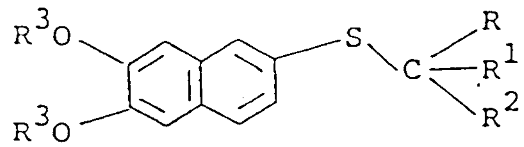

[I']

worin R, $R^1$, $R^2$ und $R^3$ wie in Anspruch 1 definiert sind, mit einem Oxidationsmittel umfaßt.

**10.** Antiallergische Zubereitung, umfassend ein Thionaphthalinderivat mit der obigen Formel (I) oder ein nicht-toxisches Salz hiervon gemäß Anspruch 1 als der wirksame Inhaltsstoff, zusammen mit einem pharmazeutisch geeigneten Träger.

**Revendications**

**1.** Un dérivé de thionaphtalène ayant la formule suivante [I], ou un sel non toxique de celui-ci :

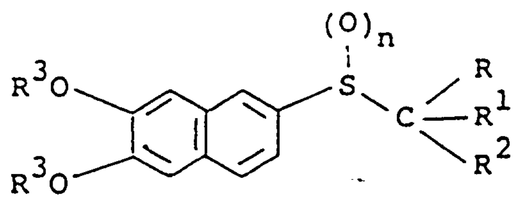

[I]

dans laquelle R représente un atome d'hydrogène, un groupe $C_1$-$C_5$ alkyle non substitué, un groupe aryle substitué ou non substitué, un groupe hétéro cyclique, un groupe

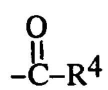

(dans lequel $R^4$ représente un groupe $C_1$-$C_4$ alkyle substitué ou non substitué, un groupe aryle substitué ou non substitué ou un groupe hétérocyclique), un groupe

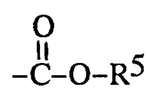

(dans lequel $R^5$ représente un atome d'hydrogène, un groupe $C_1$-$C_{10}$ alkyle substitué ou non substitué, un groupe $C_3$-$C_{10}$ alcényle substitué ou non substitué ou un groupe $C_5$-$C_7$ cycloalkyle substitué ou non substitué), un groupe

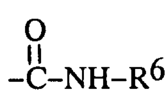

(où $R^6$ représente un groupe $C_1$-$C_4$ alkyle substitué ou non substitué, un groupe aryle substitué ou non substitué ou un groupe hétérocyclique) ou un groupe cyano,

$R^1$ et $R^2$ représentent indépendamment chacun un atome d'hydrogène, un groupe $C_1$-$C_4$ alkyle ou un groupe phényle,

$R^3$ représente un atome d'hydrogène, un groupe $C_1$-$C_4$ alkyle ou

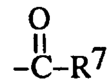

(où $R^7$ représente $-OR^{81}$, $-R^{82}$ ou $-NR^{83}{}_2$ et $R^{81}$, $R^{82}$ et $R^{83}$ représentent chacun un groupe $C_1$-$C_4$ alkyle), et

n représente un nombre entier de 0 à 2, à la condition que R, $R^1$ et $R^2$ ne peuvent pas tous représenter H lorsque n est 0.

**2.** Un dérivé de thionaphtalène ou un sel non toxique de ce dernier selon la revendication 1, selon laquelle dans la formule [I], R est un groupe

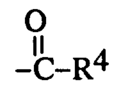

(où $R^4$ représente un groupe $C_1$-$C_5$ alkyle ou un groupe $C_6$-$C_{10}$ aryle), un groupe

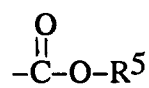

($R^5$ représente un atome d'hydrogène, un groupe $C_1$-$C_6$ alkyle qui peut être substitué par un groupe phényle, un groupe $C_3$-$C_{10}$ alcényle ou un groupe $C_5$-$C_6$ cycloalkyle).

**3.** Un dérivé de thionaphtalène ou un sel non toxique de celui-ci selon la revendication 1, selon laquelle, dans la formule [I], $R^1$ et $R^2$ représentent chacun indépendamment un atome d'hydrogène ou un groupe $C_1$-$C_4$ alkyle.

**4.** Un dérivé de thionaphtalène ou un sel non toxique de celui-ci selon la revendication 1, selon laquelle, dans la formule [I], $R^3$ est un atome d'hydrogène ou un groupe

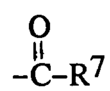

(où $R^7$ représente un groupe $C_1$-$C_4$ alkyle).

**5.** Un dérivé de thionaphtalène ou un sel non toxique de ce dernier selon la revendication 1, selon laquelle dans la formule [I], n est égal à 0.

**6.** Un dérivé de thionaphtalène ou un sel non toxique de ce dernier selon la revendication 2, selon laquelle dans la formule [I], $R^1$ et $R^2$ désignent chacun indépendamment un atome d'hydrogène ou un groupe $C_1$-$C_4$ alkyle et $R^3$ est un atome d'hydrogène ou

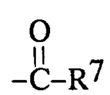

(où $R^7$ représente un groupe $C_1$-$C_4$ alkyle).

**7.** Un dérivé de thionaphtalène ou un sel non toxique de ce dernier selon la revendication 6, selon laquelle dans la formule [I], R est

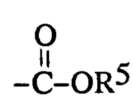

(où $R^5$ représente un atome d'hydrogène ou un groupe $C_1$-$C_5$ alkyle qui peut être substitué par un

groupe phényle.

**8.** Une méthode de préparation d'un dérivé de thionaphtalène ayant la formule [I']:

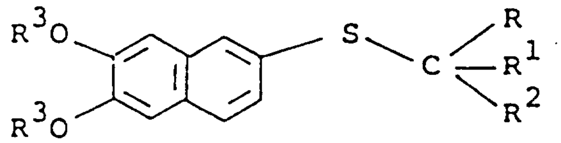

[I']

dans laquelle R, $R^1$, $R^2$ et $R^3$ sont comme définis dans la revendication 1 qui comprend la réaction d'un thiol ayant la formule [II]:

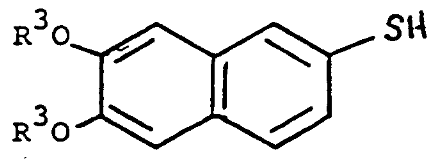

[II]

dans laquelle $R^3$ est comme défini dans la revendication 1, avec un composé halogénure ayant la formule [III]:

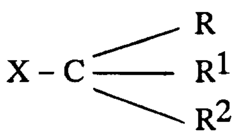

[III]

dans laquelle X représente un atome d'halogène, R, $R^1$, $R^2$ sont comme définis dans la revendication 1, en présence d'un composé basique, à la condition que R, $R^1$ et $R^2$ ne peuvent pas tous représenter H.

**9.** Une méthode de préparation d'un dérivé de thionaphtalène ayant la formule [I"] :

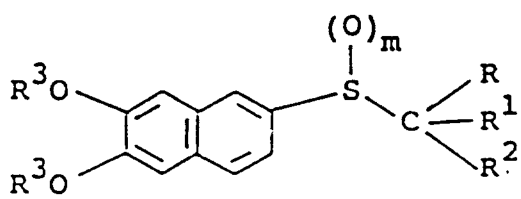

[I"]

dans laquelle R, $R^1$, $R^2$ et $R^3$ sont comme définis dans la revendication 1 et m représente 1 ou 2, qui comprend la réaction d'un dérivé de thionaphtalène ayant la formule [I']:

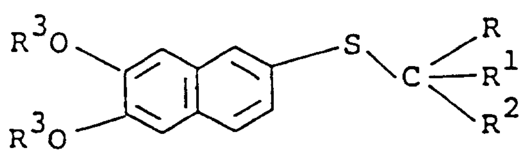

[I']

dans laquelle R, $R^1$, $R^2$ et $R^3$ sont comme définis dans la revendication 1 avec un agent oxydant.

**10.** Une préparation antiallergique comprenant un dérivé de thionaphtalène ayant la formule [I] précédente, ou un sel non toxique de celui-ci, selon la revendication 1 comme ingrédient actif, ensemble avec un support pharmaceutiquement acceptable.